# EUROPEAN PATENT SPECIFICATION

(11) **EP 3 194 393 B1**
(45) Date of publication and mention of the grant of the patent: **11.03.2020**
(21) Application number: 15767324.5
(22) Date of filing: 04.09.2015
(51) Int. Cl.: C07D 417/12, C07D 285/125, A01P 13/00, A01N 43/82

(54) **1,3,4-THIADIAZOLES HAVING A HERBICIDAL ACTIVITY, THEIR AGRONOMICAL COMPOSITIONS AND RELATIVE USE**
1,3,4-THIADIAZOLE MIT HERBIZIDER WIRKUNG, IHRE AGRONOMICAL ZUSAMMENSETZUNGEN UND ENTSPRECHENDE VERWENDUNG
1,3,4-THIADIAZOLES PRÉSENTANT UNE ACTIVITÉ HERBICIDE, LEURS COMPOSITIONS AGRONOMIQUES ET UTILISATION ASSOCIÉE

(30) Priority: 19.09.2014 IT MI20141616
(43) Date of publication of application: 26.07.2017
(73) Proprietor: ISAGRO S.p.A., 20153 Milano (IT)
(72) Inventor: MEAZZA, Giovanni, 21047 Saronno (VA) (IT); SILLANI, Laura, 28021 Borgomanero (NO) (IT); FORGIA, Daniele, 28021 Borgomanero (NO) (IT); GUSMEROLI, Marilena, 20900 Monza (MB) (IT); BONDONI, Ivan, 20010 Santo Stefano Ticino (MI) (IT)
(74) Representative: Marturano, Pasqualino
(86) International application number: PCT/IB2015/056769
(87) International publication number: WO 2016/042435

(56) References cited:
- EP-A1- 0 335 646
- WO-A1-93/09100
- CN-A- 104 530 037
- GB-A- 2 379 218
- US-A- 4 576 629
- FOROUMADI A ET AL: "Synthesis and antibacterial activity of N-(5-benzylthio-1,3,4-thiadiazol-2-yl) and N-(5-benzylsulfonyl-1,3,4-thiadiazol-2-yl) piperazinyl quinolone derivatives", BIOORGANIC & MEDICINAL CHEMISTRY LETTERS, PERGAMON, AMSTERDAM, NL, vol. 15, no. 20, 15 October 2005 (2005-10-15), pages 4488-4492, XP027801372, ISSN: 0960-894X [retrieved on 2005-10-15]

## Description

The present invention relates to new thiadiazoles having general formula (I): 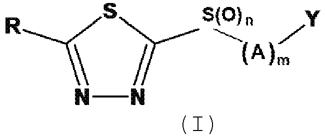 and their use as herbicides.

### DESCRIPTION

The present invention relates to new substituted thiadiazoles.

More specifically, the present invention relates to new substituted thiadiazoles having a high herbicidal activity, a process for their preparation and their use as herbicides for controlling weeds in agricultural crops.

Patent application DE 2533604 describes substituted 1,3,4-thiadiazoles having a herbicidal and insecticidal activity, such as, for example, 2-(2,6-dichlorophenylsulfonyl)-5-(trifluoromethyl)-1,3,4-thiadiazole. In this document, absolutely no mention is made of the possibility that compounds having a similar structure can also exert a high herbicidal activity with respect to weeds in agricultural crops.

The Applicant has surprisingly found that, by suitably modifying the substituents present on the thiadiazole ring, products are obtained, having a significant herbicidal activity with respect to numerous weeds. At the same time, these products have a low or zero phytotoxicity for the crops of agrarian interest and can consequently also be used as selective herbicides.

An object of the present invention therefore relates to new 1,3,4-thiadiazoles having general formula (I): 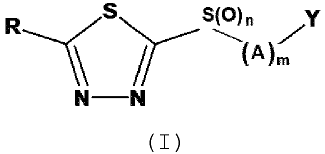 wherein R, R₁, R₂, Y, n and m have the meanings defined in claim 1.

Examples of C₁-C₄ alkyl are methyl, ethyl, n-propyl, isopropyl, n-butyl, isobutyl, sec-butyl, tert-butyl.

Examples of C₃-C₆ cycloalkyl are cyclopropyl, cyclobutyl, cyclopentyl, cyclohexyl.

Examples of C₄-C₇ cycloalkylalkyl are cyclopropylmethyl, cyclobutylmethyl, cyclopentylmethyl, cyclohexylmethyl.

Phenyl optionally substituted or naphthyl optionally substituted refers to a phenyl or naphthyl group that can have one or more substituents, equal to or different from each other, preferably selected from halogen atoms, C₁-C₄ alkyls, C₁-C₄ haloalkyls, C₁-C₄ alkoxy, C₁-C₄ haloalkoxy, C₃-C₆ cycloalkoxy, C₄-C₇ cycloalkylalkoxy, phenoxy, C₁-C₄ alkylthio, C₁-C₄ haloalkylthio, C₁-C₄ alkylsulfinyls, C₁-C₄ alkylsulfonyls, C₁-C₄ alkylamino, C₂-C₈ dialkylamino, cyano, C₂-C₅ alkoxycarbonyls, benzyloxycarbonyls, phenoxycarbonyls, or two substituents together represent a C₁-C₄ alkylenedioxy group.

An aromatic heterocyclic group optionally substituted refers to a compound with a pentatomic ring, hexatomic ring, benzocondensed or heterobicyclic, containing at least one heteroatom selected from nitrogen, oxygen and sulfur.

Said aromatic heterocyclic group can have one or more substituents, equal to or different from each other, preferably selected from halogen atoms, C₁-C₄ alkyls, C₁-C₄ haloalkyls, C₁-C₄ alkoxy, C₁-C₄ haloalkoxy, C₃-C₆ cycloalkoxy, C₄-C₇ cycloalkylalkoxy, phenoxy, C₁-C₄ alkylthio, C₁-C₄ haloalkylthio, C₁-C₄ alkylsulfinyls, C₁-C₄ alkylsulfonyls, C₁-C₄ alkylamino, C₂-C₈ dialkylamino, cyano, nitro, carboxyl, C₂-C₅ alkoxycarbonyls, benzyloxycarbonyls, phenoxycarbonyls, or two substituents together represent a C₁-C₄ alkylenedioxy group.

Examples of an aromatic heterocyclic group are: pyridyl, pyridyl N-oxide, pyrimidyl, pyridazyl, pyrazyl, furyl, thienyl, pyrrolyl, oxazolyl, thiazolyl, isoxazolyl, isothiazolyl, oxadiazolyl, thiadiazolyl, pyrazolyl, imidazolyl, triazolyl, indolyl, benzofuranyl, benzothiophenyl, benzoxazolyl, benzothiazolyl, benzoxadiazolyl, benzothiadiazolyl, benzopyrazolyl, benzimidazolyl, benzotriazolyl, triazolopyridyl, triazole-pyrimidyl, thiazole-triazolyl.

Compounds according to the invention having general formula (I) which are interesting for their herbicidal activity are compounds wherein R, R₁, R₂, Y, n and m have the meanings specified in Table 1:

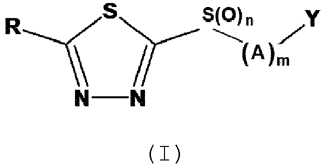

**Table 1**

| **Nr.** | **R** | **R₁** | **R₂** | **Y** | **n** | **m** |
|---|---|---|---|---|---|---|
| 1 | CH₃ | H | H | 2,6-diCl-phenyl | 1 | 1 |
| 2 | CH₃ | H | H | 2,6-diCl-phenyl | 2 | 1 |
| 3 | CH₃ | H | H | 2,6-diF-phenyl | 1 | 1 |
| 4 | CH₃ | H | H | 2,6-diF-phenyl | 2 | 1 |
| 5 | CH₃ | H | H | 2-Cl-6-F-phenyl | 1 | 1 |
| 6 | CH₃ | H | H | 2-Cl-6-F-phenyl | 2 | 1 |
| 7 | CH₃ | H | H | 2,4-diCl-phenyl | 1 | 1 |
| 8 | CH₃ | H | H | 2,4-diCl-phenyl | 2 | 1 |
| 9 | CH₃ | H | H | 1-CH₃-3-CF₃-5-OCH₂CF₃-pyrazol-4-yl | 1 | 1 |
| 10 | CH₃ | H | H | 1-CH₃-3-CF₃-5-OCH₂CF₃-pyrazol-4-yl | 2 | 1 |
| 11 | CH₃ | H | H | 2-Cl-4,5-methyl-endioxy-phenyl | 1 | 1 |
| 12 | CH₃ | H | H | 2-Cl-4,5-methyl-endioxy-phenyl | 2 | 1 |
| 13 | CH₃ | H | H | 2,5-diCl-4-OEt-phenyl | 1 | 1 |
| 14 | CH₃ | H | H | 2,5-diCl-4-OEt-phenyl | 2 | 1 |
| 17 | CH₃ | H | H | 2-CH₃-phenyl | 1 | 1 |
| 18 | CH₃ | H | H | 2-CH₃-phenyl | 2 | 1 |
| 19 | CH₃ | H | H | 2,5-diCl-4-OMe-phenyl | 1 | 1 |
| 20 | CH₃ | H | H | 2,5-diCl-4-OCF₂CF₂H-phenyl | 2 | 1 |
| 21 | CH₃ | H | H | 2,5-diCl-4-OCF₂CF₂H-phenyl | 1 | 1 |
| 22 | CH₃ | H | H | 2,5-diCl-4-OMe-phenyl | 2 | 1 |
| 23 | CH₃ | H | H | 2,5-diCH₃-phenyl | 1 | 1 |
| 24 | CH₃ | H | H | 2,5-diCH₃-phenyl | 2 | 1 |
| 25 | CH₃ | H | H | 2,6-diOCH₃-phenyl | 1 | 1 |
| 26 | CH₃ | H | H | 2,6-diOCH₃-phenyl | 2 | 1 |
| 27 | CH₃ | H | H | 3-CF₃-phenyl | 1 | 1 |
| 28 | CH₃ | H | H | 2-CF₃-phenyl | 2 | 1 |
| 29 | CH₃ | H | H | 2,3,4,5,6-pentaF-phenyl | 1 | 1 |
| 30 | CH₃ | H | H | 2,3,4,5,6-pentaF-phenyl | 2 | 1 |
| 31 | CH₃ | H | H | 2-SO₂Me-5-COCH₃-6-Cl-phenyl | 1 | 1 |
| 32 | CH₃ | H | H | 2-SO₂Me-5-COCH₃-6-Cl-phenyl | 2 | 1 |
| 33 | CH₃ | H | H | 2,6-diCl-5-COCH₃-phenyl | 1 | 1 |
| 34 | CH₃ | H | H | 2,6-diCl-5-COCH₃-phenyl | 2 | 1 |
| 35 | CH₃ | H | H | 2-CH₃-5-Br-thiazol-2-yl | 1 | 1 |
| 36 | CH₃ | H | H | 2-CH₃-5-Br-thiazol-2-yl | 2 | 1 |
| 37 | CH₃ | H | H | 3-COOEt-furan-2-yl | 1 | 1 |
| 38 | CH₃ | H | H | 3-COOEt-furan-2-yl | 2 | 1 |
| 39 | CH₃ | H | H | 3*-*tBu-isoxazol-2-yl | 1 | 1 |
| 40 | CH₃ | H | H | 3-*t*Bu-isoxazol-2-yl | 2 | 1 |
| 41 | CH₃ | H | H | 6-Cl-pyiridin-3-yl | 1 | 1 |
| 42 | CH₃ | H | H | 6-Cl-pyridin-3-yl-N-oxide | 2 | 1 |
| 43 | CH₃ | H | H | 6-Cl-pyridin-2-yl | 1 | 1 |
| 44 | CH₃ | H | H | 6-Cl-pyridin-2-yl-N-oxide | 2 | 1 |
| 45 | CH₃ | H | H | pyridin-3-yl | 1 | 1 |
| 46 | CH₃ | H | H | pyridin-3-yl-N-oxide | 2 | 1 |
| 47 | CH₃ | H | H | pyridin-2-yl | 1 | 1 |
| 48 | CH₃ | H | H | pyridin-2-yl-N-oxide | 2 | 1 |
| 49 | CH₃ | H | CH₃ | 2,4-diCl-phenyl | 1 | 1 |
| 50 | CH₃ | H | CH₃ | 2,4-diCl-phenyl | 2 | 1 |
| 51 | CH₃ | H | H | 1-CH₃-3-CF₃-5-OCF₂H-pyrazol-4-yl | 1 | 1 |
| 52 | CH₃ | H | H | 1-CH₃-3-CF₃-5-OCF₂H-pyrazol-4-yl | 2 | 1 |
| 53 | CH₃ | H | H | 1-CH₃-3-CF₂H-pyrazol-4-yl | 1 | 1 |
| 54 | CH₃ | H | H | 1-CH₃-3-CF₂H-pyrazol-4-yl | 2 | 1 |
| 55 | CH₃ | H | H | 4-F-phenyl | 1 | 1 |
| 56 | CH₃ | H | H | 4-F-phenyl | 2 | 1 |
| 57 | CH₃ | H | H | 1-CH₃-3-CF₃-5-OCF₂H-pyrazol-4-yl | 1 | 2 |
| 58 | CH₃ | H | H | 1-CH₃-3-CF₃-5-OCF₂H-pyrazol-4-yl | 2 | 2 |
| 59 | CH₃ | H | H | 1-CH₃-3-CF₃-5-OCF₂H-pyrazol-4-yl | 1 | 3 |
| 60 | CH₃ | H | H | 1-CH₃-3-CF₃-5-OCF₂H-pyrazol-4-yl | 2 | 3 |
| 61 | CH₃ | - | - | 1-CH₃-3-CF₃-5-OCF₂H-pyrazol-4-yl | 1 | 0 |
| 62 | CH₃ | - | - | 1-CH₃-3-CF₃-5-OCF₂H-pyrazol-4-yl | 2 | 0 |
| 63 | CH₃ | H | CH₃ | 1-CH₃-3-CF₃-5-OCF₂H-pyrazol-4-yl | 1 | 1 |
| 64 | CH₃ | H | CH₃ | 1-CH₃-3-CF₃-5-OCF₂H-pyrazol-4-yl | 2 | 1 |
| 65 | CH₃ | H | CH₃ | 1-CH₃-3-CF₃-5-OCF₂H-pyrazol-4-yl | 1 | 2 |
| 66 | CH₃ | H | CH₃ | 1-CH₃-3-CF₃-5-OCF₂H-pyrazol-4-yl | 2 | 2 |
| 67 | CH₃ | H | C₃H₅ | 1-CH₃-3-CF₃-5-OCF₂H-pyrazol-4-yl | 1 | 1 |
| 68 | CH₃ | H | C₃H₅ | 1-CH₃-3-CF₃-5-OCF₂H-pyrazol-4-yl | 2 | 1 |
| 69 | CH₃ | H | H | 2,6-diCl-phenyl | 1 | 3 |
| 70 | CH₃ | H | H | 2,6-diCl-phenyl | 2 | 3 |
| 71 | CH₃ | H | H | 2,6-diCl-phenyl | 1 | 2 |
| 72 | CH₃ | H | H | 2,6-diCl-phenyl | 2 | 2 |
| 73 | CH₃ | - | - | 2,6-diCl-phenyl | 1 | 0 |
| 74 | CH₃ | - | - | 2,6-diCl-phenyl | 2 | 0 |
| 75 | CH₃ | H | CH₃ | 2,6-diCl-phenyl | 1 | 1 |
| 76 | CH₃ | H | CH₃ | 2,6-diCl-phenyl | 2 | 1 |
| 77 | CH₃ | H | CH₃ | 2,6-diCl-phenyl | 1 | 2 |
| 78 | CH₃ | H | CH₃ | 2,6-diCl-phenyl | 2 | 2 |
| 79 | CH₃ | CH₃ | CH₃ | 2,6-diCl-phenyl | 1 | 1 |
| 80 | CH₃ | CH₃ | CH₃ | 2,6-diCl-phenyl | 2 | 1 |
| 81 | CH₃ | H | C₃H₅ | 2,6-diCl-phenyl | 1 | 1 |
| 82 | CH₃ | H | C₃H₅ | 2,6-diCl-phenyl | 2 | 1 |
| 83 | C₂H₅ | H | H | 2,6-diCl-phenyl | 1 | 1 |
| 84 | C₂H₅ | H | H | 2,6-diCl-phenyl | 2 | 1 |
| 85 | C₃H₅ | H | H | 2,6-diCl-phenyl | 1 | 1 |
| 86 | C₃H₅ | H | H | 2,6-diCl-phenyl | 2 | 1 |
| 87 | C₆H₁₁ | H | H | 2,6-diCl-phenyl | 1 | 1 |
| 88 | C₆H₁₁ | H | H | 2,6-diCl-phenyl | 2 | 1 |
| 89 | C₆H₁₁CH₂ | H | H | 2,6-diCl-phenyl | 1 | 1 |
| 90 | C₆H₁₁CH₂ | H | H | 2,6-diCl-phenyl | 2 | 1 |
| 91 | C₃H₅CH₂ | H | H | 2,6-diCl-phenyl | 1 | 1 |
| 92 | C₃H₅CH₂ | H | H | 2,6-diCl-phenyl | 2 | 1 |
| 93 | CH₃ | CH₃ | CH₃ | 1-CH₃-3-CF₃-5-OCF₂H-pyrazol-4-yl | 1 | 1 |
| 94 | CH₃ | CH₃ | CH₃ | 1-CH₃-3-CF₃-5-OCF₂H-pyrazol-4-yl | 2 | 1 |
| 95 | C₂H₅ | H | H | 1-CH₃-3-CF₃-5-OCF₂H-pyrazol-4-yl | 1 | 1 |
| 96 | C₂H₅ | H | H | 1-CH₃-3-CF₃-5-OCF₂H-pyrazol-4-yl | 2 | 1 |
| 97 | C₃H₅ | H | H | 1-CH₃-3-CF₃-5-OCF₂H-pyrazol-4-yl | 1 | 1 |
| 98 | C₃H₅ | H | H | 1-CH₃-3-CF₃-5-OCF₂H-pyrazol-4-yl | 2 | 1 |
| 99 | C₆H₁₁ | H | H | 1-CH₃-3-CF₃-5-OCF₂H-pyrazol-4-yl | 1 | 1 |
| 100 | C₆H₁₁ | H | H | 1-CH₃-3-CF₃-5-OCF₂H-pyrazol-4-yl | 2 | 1 |
| 101 | C₆H₁₁CH₂ | H | H | 1-CH₃-3-CF₃-5-OCF₂H-pyrazol-4-yl | 1 | 1 |
| 102 | C₆H₁₁CH₂ | H | H | 1-CH₃-3-CF₃-5-OCF₂H-pyrazol-4-yl | 2 | 1 |
| 103 | C₃H₅CH₂ | H | H | 1-CH₃-3-CF₃-5-OCF₂H-pyrazol-4-yl | 1 | 1 |
| 104 | C₃H₅CH₂ | H | H | 1-CH₃-3-CF₃-5-OCF₂H-pyrazol-4-yl | 2 | 1 |
| 105 | CH₃ | H | H | 2-CF₃-4-F-phenyl | 1 | 1 |
| 106 | CH₃ | H | H | 2-CF₃-4-F-phenyl | 2 | 1 |
| 107 | CH₃ | H | H | 2-CF₃-5-F-phenyl | 1 | 1 |
| 108 | CH₃ | H | H | 2-CF₃-5-F-phenyl | 2 | 1 |
| 109 | CH₃ | H | H | 2-CF₃-6-F-phenyl | 1 | 1 |
| 110 | CH₃ | H | H | 2-CF₃-6-F-phenyl | 2 | 1 |
| 111 | CH₃ | H | H | 5-CH₃-1,3,4-thiadiazol-4-yl | 1 | 1 |
| 112 | CH₃ | H | H | 5-CH₃-1,3,4-thiadiazol-4-yl | 2 | 1 |
| 113 | CH₃ | H | H | 2-CH₃-4-CF₃-1,3-thiazol-5-yl | 1 | 1 |
| 114 | CH₃ | H | H | 2-CH₃-4-CF₃-1,3-thiazol-5-yl | 2 | 1 |
| 115 | CH₃ | H | H | 1-CH₃-3-CF₃-5-Cl-pyrazol-4-yl | 1 | 1 |
| 116 | CH₃ | H | H | 1-CH₃-3-CF₃-5-Cl-pyrazol-4-yl | 2 | 1 |
| 117 | *i*C₃H₇ | H | H | 2,6-diCl-phenyl | 1 | 1 |
| 118 | *i*C₃H₇ | H | H | 2,6-diCl-phenyl | 2 | 1 |
| 119 | *i*C₃H₇ | H | H | 2,5-diCl-4-OEt-phenyl | 1 | 1 |
| 120 | *i*C₃H₇ | H | H | 2,5-diCl-4-OEt-phenyl | 2 | 1 |
| 121 | C₂H₅ | H | H | 1-CH₃-3-CF₃-5-OCH₂CF₃-pyrazol-4-yl | 1 | 1 |
| 122 | C₂H₅ | H | H | 1-CH₃-3-CF₃-5-OCH₂CF₃-pyrazol-4-yl | 2 | 1 |
| 123 | Cl | H | H | 1-CH₃-3-CF₃-5-OCH₂CF₃-pyrazol-4-yl | 1 | 1 |
| 124 | Cl | H | H | 1-CH₃-3-CF₃-5-OCH₂CF₃-pyrazol-4-yl | 2 | 1 |

Particularly preferred are compounds having general formula (I) wherein R, R₁, R₂, Y, n and m have the following meanings:

| **Nr.** | **R** | **R₁** | **R₂** | **Y** | **n** | **m** |
|---|---|---|---|---|---|---|
| 1 | CH₃ | H | H | 2,6-diCl-phenyl | 1 | 1 |
| 51 | CH₃ | H | H | 1-CH₃-3-CF₃-5-OCF₂H-pyrazol-4-yl | 1 | 1 |
| 9 | CH₃ | H | H | 1-CH₃-3-CF₃-5-OCH₂CF₃-pyrazol-4-yl | 1 | 1 |
| 83 | C₂H₅ | H | H | 2,6-diCl-phenyl | 1 | 1 |
| 95 | C₂H₅ | H | H | 1-CH₃-3-CF₃-5-OCF₂H-pyrazol-4-yl | 1 | 1 |
| 121 | C₂H₅ | H | H | 1-CH₃-3-CF₃-5-OCH₂CF₃-pyrazol-4-yl | 1 | 1 |

As is evident to a skilled person in the field, the compounds having general formula (I), for particular meanings of the substituents R₁, R₂ and when n has the value of 1, can be obtained in the form of two or more optical isomers.

An object of the present invention therefore relates to compounds having general formula (I) wherein R, R₁, R₂, Y, n and m have the meanings defined in claim 1 in racemic form, isomerically pure, or mixtures thereof, possibly obtained during the preparation of compounds having general formula (I) or deriving from an incomplete separation of the same isomers, in any proportion.

A further object of the present invention relates to a process for the preparation of the compounds having general formula (I) wherein R, R₁, R₂, Y, n and m have the meanings defined in claim 1.

In particular, the compounds having general formula (I) wherein R, R₁, R₂, Y, n and m have the meanings defined in claim 1 can be prepared from the corresponding thioether having general formula (II) by oxidation of the sulfur atom, as indicated in reaction scheme 1.

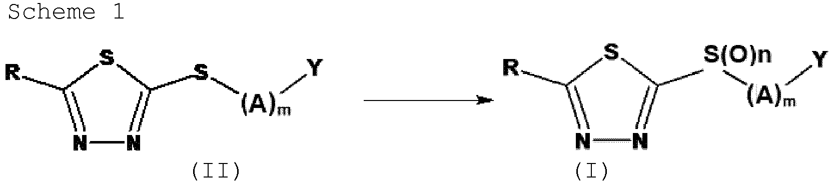

The reaction, as described for example in WO 02/062770, envisages the use of at least one oxidizing agent in at least one solvent. Among the oxidizing agents, organic peroxides, such as 4-chloro-perbenzoic acid, peracetic acid or inorganic peroxides such as, for example, hydrogen peroxide, potassium permanganate, sodium periodate, can be used.

The solvents that can be used are preferably selected from: halogenated hydrocarbons such as dichloromethane or dichloroethane or chloroform, ethers such as dioxane or tetrahydrofuran, amides such as N,N-dimethylformamide or N-methyl-pyrrolidone, alcohols such as methanol, ethanol, propanol, isopropanol or ketones such as acetone or 2-butanone, acetic acid, water and mixtures thereof.

The reaction is carried out at a temperature ranging from 0 to 60°C, for a time ranging from 1 to 72 hours.

The compound having formula (II) can be prepared as indicated in scheme 2, according to what is described in WO 03/037878.

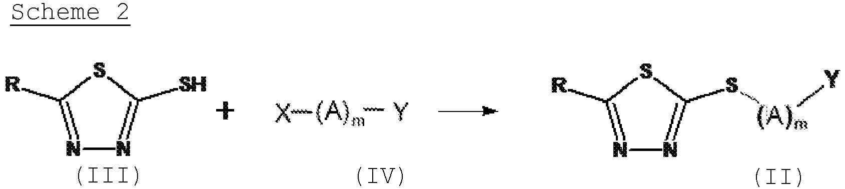

The reaction envisages the treatment of the compound having formula (III) with a compound having formula (IV), wherein X represents an outgoing group, such as, for example, a halogen selected from Cl, Br, I, or a p-toluenesulfonate or trifluoromethanesulfonate group, in the presence of an organic or inorganic base such as, for example, triethylamine, pyridine, sodium acetate, potassium or calcium bicarbonate, sodium or potassium hydroxide, in a suitable solvent such as dichloroethane, chloroform or methylene chloride at a temperature ranging from room temperature to the reflux temperature of the solvent selected.

The compound having formula (II), when m has the value 0, can be prepared by reacting a compound having formula (III) with a compound having formula (IV) wherein m=0 in the presence of a base, preferably sodium or potassium carbonate or a metal hydride, in a solvent such as N,N-dimethylformamide, toluene, xylene at a temperature ranging from room temperature to 140°C, in the presence or absence of a palladium, copper or nickel catalyst as described, for example, in "Tetrahedron", 61 (2005), pages 5253-5259.

The compounds having formula (III), when they are not commercial products, can be easily obtained according to methods known in organic chemistry, as described, for example, in US 5162539 or in US 7238689.

The compounds having formula (IV), when they are not commercial products, can be obtained according to what is described in US 20050215797 as indicated in scheme 3 wherein X represents a halogen atom.

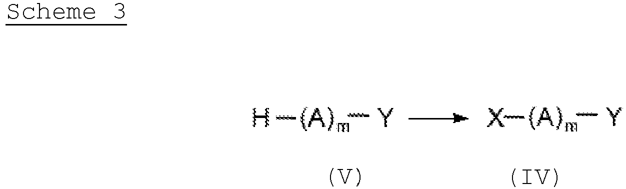

Alternatively, the compounds having formula (IV), when X represents a p-toluenesulfonate or trifluoromethanesulfonate group, can be obtained from the corresponding alcohols having formula (VI) according to what is described in Theodora W. Greene "Protective Groups in Organic Synthesis" Third Edition pages 198-199 and indicated in scheme 4.

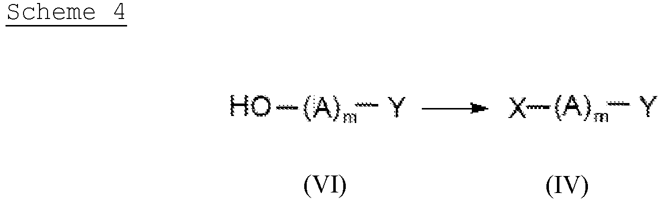

When R₁ and R₂ have the meaning of hydrogen, the compounds having formula (VI) can be easily obtained by reduction of the corresponding acid or corresponding aldehyde according to what is described in "Tetrahedron", 52 (1996), pages 12137-12158 or in WO 2006123088.

Alternatively, the compound having formula (II) can be prepared by treatment of a compound having formula (VII), with X having the meaning of halogen, such as, for example, chlorine or bromine, with a salt having formula (VIII), as indicated in reaction scheme 5.

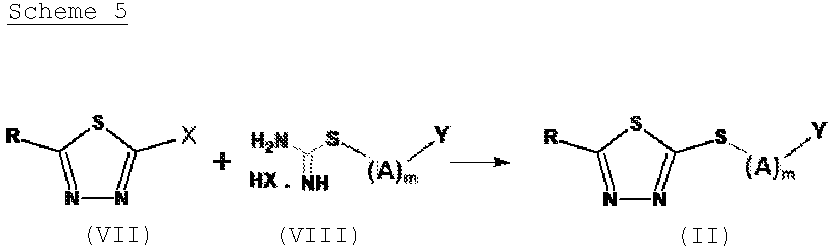

The reaction envisages an in situ hydrolysis of the salt having formula (VIII) in a solvent such as, for example, dioxane, tetrahydrofuran, dichloromethane, chloroform, toluene or in a mixture thereof with water in the presence or absence of an inorganic base such as sodium or potassium carbonate, sodium or potassium bicarbonate, sodium or potassium hydroxide, at room temperature, to give the corresponding thiol and subsequent treatment with a compound having formula (VII), according to what is described in US 20050215797.

If desired, the thiol obtained by the hydrolysis of (VIII), can be possibly isolated and treated with (VII) in a subsequent passage.

As indicated, the compounds having general formula (I) have a high herbicidal activity which makes them suitable for use in the agrarian field in the defense of useful crops from weeds.

A further object of the present invention therefore relates to the use of thiadiazoles having general formula (I) in racemic form, isomerically pure or mixtures thereof or a composition containing them, as herbicides according to claim 7.

In particular, the compounds object of the present invention, are effective in the control, in both pre-emergence and post-emergence of numerous weeds, in particular monocotyledonous and dicotyledonous weeds.

At the same time, said compounds can show compatibility or the absence of toxic effects with respect to crops useful in pre- and/or post-emergence treatment.

The compounds of the present invention can therefore act either as total herbicides or selective herbicides, also in relation to the quantity of active principle used.

Examples of weeds that can be effectively controlled using the compounds having general formula (I) are: Abutilon theofrasti, Alisma plantago, Amaranthus spp., Amni maius, Capsella bursa pastoris, Chenopodium album, Convolvulus sepium, Galium aparine, Geranium dissectum, Heteranthera spp., Ipomea spp., Matricaria spp., Papaver rhoaes, Phaseolus aureus, Polygonum persicaria, Portulaca oleracea, Setaria viridis, Sida spinosa, Sinapsis arvensis, Solanum nigrum, Stellaria media, Veronica spp., Viola spp., Xanthium spp., Alopecurus myosuroides, Anisanta spp., Apera spica-venti, Avena spp., Cyperus spp., Digitaria sanguinalis, Eleusine spp. Echinochloa spp., Eleocharis avicularis, Lolium spp., Panicum spp., Poa spp., Scirpus spp., Sorghum spp., etc.

At the doses of use suitable for agrarian applications, many of the above compounds have not shown any toxic effects towards one or more important agrarian crops such as wheat (Triticum sp.), barley (Hordeum vulgare), corn (Zea mays), soybean (Glycine max) .

A further object of the present invention relates to a method for controlling weeds in cultivated areas according to claim 10, which comprises the application of an effective dose of at least one compound having general formula (I) or a composition containing it, to the agricultural crops of interest.

The quantity of compound to be applied for obtaining the desired effect (effective dose) can vary in relation to various factors such as, for example, the compound used, the crop to be preserved, the weed to be fought, the degree of infestation, the climatic conditions, the characteristics of the soil, the application method, etc.

Doses of compound ranging from 1 g to 1,000 g per hectare generally provide a sufficient control.

For applying at least one compound having general formula (I) as herbicide on a weed or crop, said compound having general formula (I) is formulated in the form of a composition which comprises, in addition to the above compound, a solvent medium and/or a suitable inert diluent, said inert diluent being either in solid or liquid form, possibly in the presence of at least one agronomically acceptable excipient.

A further object of the present invention therefore relates to a herbicidal composition according to claim 3, comprising at least one compound having general formula (I) in racemic form, isomerically pure or mixtures thereof, at least one solvent and/or inert diluent, solid or liquid, and at least one agronomically acceptable excipient.

Kaolin, alumina, silica, talc, bentonite, gypsum, quartz, dolomite, attapulgite, montmorillonite, diatomaceous earth, cellulose, starch, etc.., can be used as inert solid diluents, or carriers.

Inert liquid diluents which can be used, are water or organic solvents such as aromatic hydrocarbons (xylols, blends of alkylbenzenes, etc.), aliphatic hydrocarbons (hexane, cyclohexane, etc.), halogenated aromatic hydrocarbons (chlorobenzene, etc.), alcohols (methanol, propanol, butanol, octanol, etc.), esters (isobutyl acetate, etc.), ketones (acetone, cyclohexanone, acetophenone, isophorone, ethylamylketone, etc.), or vegetable or mineral oils or mixtures thereof, etc..

Said at least one agronomically acceptable excipient is preferably selected from surfactants, dispersants and stabilizers.

Surfactants which can be used are wetting and emulsifying agents, of the non-ionic type (polyethoxylated alkyl phenols, polyethoxylated fatty alcohols, etc.), of the anionic type (alkylbenzenesulfonates, alkylsulfonates, etc.), of the cationic type (alkyl ammonium quaternary salts, etc.).

Dispersing agents can also be added (for example lignin and its salts, cellulose derivatives, alginates, etc.), stabilizers (for example antioxidants, UV absorbers, etc.).

Among the various formulations suitable for agricultural use, dry powders, wettable powders, emulsifiable concentrates, micro-emulsions, pastes, granulates, solutions, suspensions, etc., can be mentioned: the selection of the type of composition depends on the specific use.

In order to extend the spectrum of activity of the above compositions, one or more other active ingredients compatible with the compounds having general formula (I), can be added such as, for example, herbicides other than the compounds having general formula (I), fungicides, insecticides, acaricides, fertilizers, safeners, etc..

Examples of herbicides other than the compounds having general formula (I) which can be added to the compositions containing at least one compound having general formula (I) in order to broaden its spectrum of activity and possibly create synergistic compositions, are the following:
acetochlor, acifluorfen, aclonifen, alachlor, ametryn, amicarbazone, amidosulfuron, amino-cyclopyrachlor, aminopyralid, amitrole, anilofos, asulam, atrazine, azafenidin, azimsulfuron, aziprotryne, beflubutamid, benazolin, bencarbazone, benfluralin, benfuresate, bensulide, bentazone, benzfendizone, benzobicyclon, benzofenap, benzthiazuron, bicyclopyrone, bifenox, bilanafos, bromacil, bromobutide, bromofenoxim, bromoxynil, butachlor, butafenacil, butamifos, butenachlor, butralin, butroxydim, butylate, cafenstrole, carbetamide, carfentrazone-ethyl, chlomethoxyfen, chloramben, chlorbromuron, chlorbufam, chlorflurenol, chloridazon, chlornitrofen, chlorotoluron, chloroxuron, chlorpropham, chlorsulfuron, chlorthal, chlorthiamid, cinidon ethyl, cinmethylin, cinosulfuron, clacyfos, clethodim, clodinafop, clomazone, clomeprop, clopyralid, cloransulam-methyl, cumyluron, cyanazine, cycloate, cyclopyrimorate, cyclosulfamuron, cycloxydim, cyhalofop-butyl, cyprosulfamide, 2,4-D, 2,4-DB, daimuron, dalapon, desmedipham, desmetryn, dicamba, dichlobenil, dichlorprop, dichlorprop-P, diclofop, diclosulam, diethatyl, difenoxuron, difenzoquat, diflufenican, diflufenzopyr, dimefuron, dimepiperate, dimethachlor, dimethametryn, dimethenamid, dimethenamid-P dinitramine, dinoseb, dinoseb acetate, dinoterb, diphenamid, dipropetryn, diquat, dithiopyr, 1-diuron, eglinazine, endothal, EPTC, esprocarb, ethalfluralin, ethametsulfuron-methyl, ethidimuron, ethiozin, ethofumesate, ethoxyfen-ethyl, ethoxy-sulfuron, etobenzanid, fenoxaprop, fenoxaprop-P, fenoxasulfone, fenquinotrione, fentrazamide, fenuron, flamprop, flamprop-M, flazasulfuron, florasulam, fluazifop, fluazifop-P, fluazolate, flucarbazone-sodium, flucetosulfuron, fluchloralin, flufenacet, flufenpyr ethyl, flumetsulam, flumiclorac-pentyl, flumioxazin, flumipropin, fluometuron, fluoroglycofen, fluoronitrofen, flupoxam, flupropanate, flupyr-sulfuron, flurenol, fluridone, flurochloridone, fluroxypyr, flurtamone, fluthiacet-methyl, fomesafen, foramsulfuron, fosamine, furyloxyfen, glufosinate, glufosinate-P, glyphosate, halauxifen, halosulfuron-methyl, haloxyfop, haloxyfop-P-methyl, hexazinone, imazamethabenz, imazamox, imazapic, imazapyr, imazaquin, imazethapyr, imazosulfuron, indanofan, indaziflam, iodosulfuron, iofensulfuron, ioxynil, ipfencarbazone, isopropalin, isoproturon, isouron, isoxaben, isoxachlortole, isoxaflutole, isoxapyrifop, lactofen, lenacil, linuron, MCPA, MCPA-thioethyl, MCPB, mecoprop, mecoprop-P, mefenacet, mesosulfuron, mesotrione, metamifop, metamitron, metazachlor, metazosulfuron, methabenzthiazuron, methazole, methiozolin, methoprotryne, methyldymron, metobenzuron, metobromuron, metolachlor, S-metolachlor, metosulam, metoxuron, metribuzin, metsulfuron, molinate, monalide, monolinuron, naproanilide, napropamide, napropamide-M, naptalam, neburon, nicosulfuron, nipyraclofen, norflurazon, orbencarb, orthosulfamuron, oryzalin, oxadiargyl, oxadiazon, oxasulfuron, oxaziclomefone, oxyfluorfen, paraquat, pebulate, pendimethalin, penoxsulam, pentanochlor, pentoxazone, pethoxamid, phenmedipham, picloram, picolinafen, piperophos, pretilachlor, primisulfuron, prodiamine, profluazol, profoxydim, proglinazine, prometon, prometryne, propachlor, propanil, propaquizafop, propazine, propham, propisochlor, propoxycarbazone, propyrisulfuron, propyzamide, prosulfocarb, prosulfuron, pyraclonil, pyraflufen-ethyl, pyrasulfotole, pyrazogyl, pyrazolynate, pyrazosulfuron, pyrazoxyfen, pyribenzoxim, pyributicarb, pyridafol, pyridate, pyriftalid, pyriminobac-methyl, pyrimisulfan, pyrithiobac-sodium, pyroxasulfone, pyroxsulam, quinclorac, quinmerac, quizalofop, quizalofop-P, quizalofop-P-tefuryl, rimsulfuron, saflufenacil, sethoxydim, siduron, simazine, simetryn, sulcotrione, sulfentrazone, sulfometuron-methyl, sulfosulfuron-methyl, 2,3,6-TBA, TCA-sodium, tebutam, tebuthiuron, tefuryltrione, tembotrione, tepraloxydim, terbacil, terbumeton, terbuthyl-azine, terbutryn, thenylchlor, thiazafluron, thiazopyr, thidiazimin, thiencarbazone, thifensulfuron-methyl, thiobencarb, tiafenacil, tiocarbazil, tioclorim, tolpyralate, topramezone, tralkoxydim, triafamone, triallate, triasulfuron, triaziflam, tribenuron-methyl, triclopyr, trietazine, trifloxysulfuron, trifludimoxazin, trifluralin, triflusulfuron-methyl, tritosulfuron, vernolate.

Examples of safeners that can be added to the compositions containing at least one compound having general formula (I) are the following: benoxacor, cloquintocet-mexyl, fenclorim, mefenpir-diethyl.

The concentration of active substance, i.e. the concentration of the compound having general formula (I) in the above compositions can vary within a wide range, depending on the active compound, the applications to which they are destined, the environmental conditions and the type of formulation adopted. In general, the concentration of active substance preferably ranges from 1 to 90%.

Some examples are now provided for illustrative and non-limiting purposes of the present invention.

### EXPERIMENTAL PART

### EXAMPLE 1

### Preparation of 2-[(2,6-dichlorobenzyl)-sulfinyl]-5-methyl-1,3,4-thiadiazole [Compound Nr.1].

### a) Preparation of (2-[(2,6-dichlorobenzyl)thio]-5-methyl-1,3,4-thiadiazole [thioether having general formula (II)]

10.5 ml (75.6 mmoles) of triethylamine were added dropwise at room temperature to a suspension under nitrogen atmosphere of l0g (75.6 mmoles) of 5-methyl-1,3,4-thiadiazole-2-thiol in 40 ml of chloroform; 14.8g (75.6 mmoles) of 1,3-dichloro-2-(chloromethyl)benzene, dissolved in 10 ml of chloroform were then added. Finally, additional 15.7 ml (0.11 moles) of triethylamine were added dropwise.

The mixture was left under stirring at room temperature for a night. After control in GC-MS and LC-MS, the mixture was diluted with water and the phases were then separated; the aqueous phase was re-extracted twice with dichloromethane. The organic phases joined together, were washed with water and a saturated solution of sodium chloride.

After anhydrification on sodium sulfate, filtration and evaporation of the solvent at reduced pressure, 19.8 g (68.0 mmoles) of the desired product were obtained, as a yellow oil. Yield 90.0% LC-MS [M+H] = 292.

### b) Preparation of 2-[(2,6-dichlorobenzyl)-sulfinyl]-5-methyl-1,3,4-thiadiazole [Compound Nr.1]

4.2 g (18.92 mmoles) of 4-Cl-perbenzoic acid at 77% were added to 5 g (17.2 mmoles) of 2-[(2,6-dichlorobenzyl)thio]-5-methyl-1,3,4-thiadiazole, dissolved in 85 ml of chloroform, maintaining a temperature of about 4-5°C with an ice bath, the mixture was then left under stirring at room temperature for a night.

After control in LC-MS, the mixture was diluted with water and the phases were then separated; the aqueous phase was re-extracted twice with dichloromethane. The organic phases joined together, were washed with an aqueous solution at 5% of NaHSO₃, a saturated solution of NaHCO₃, water and a saturated solution of NaCl.

After anhydrification on sodium sulfate, filtration and evaporation of the solvent at reduced pressure, 4.9 g (15.9 mmoles) of the desired product were obtained, as a yellow oil. The raw product thus obtained was crushed with ethyl ether, filtered and dried in the air, obtaining 4.2 g (13.7 mmoles) of the desired product as a white solid. Yield 79.4%.
M.P. = 115-118°C
LC-MS [M+H] = 308.

### EXAMPLE 2

### Preparation of 2-[(2,6-dichlorobenzyl)-sulfonyl]-5-methyl-1,3,4-thiadiazole [Compound Nr.2]

11.6 g (51.6 mmoles) of 4-Cl-perbenzoic acid at 77% were added to 5 g (17.2 mmoles) of 2-[(2,6-dichlorobenzyl)thio]-5-methyl-1,3,4-thiadiazole, dissolved in 85 ml of chloroform, maintaining a temperature of about 4-5°C with an ice bath. The mixture was then left under magnetic stirring at room temperature for a night.

After control in LC-MS, the mixture was diluted with water and the phases were then separated; the aqueous phase was re-extracted twice with dichloromethane. The organic phases joined together, were washed with an aqueous solution at 5% of NaHSO₃, a saturated solution of NaHCO₃, water and a saturated solution of NaCl.

After anhydrification on sodium sulfate, filtration and evaporation of the solvent at reduced pressure, 5.1 g of yellow oil (15.8 mmoles) were obtained. The raw product thus obtained was crushed with ethyl ether, filtered and dried in the air, obtaining 4.7 g (14.7 mmoles) of the desired product as a white solid.
Yield 85.7%.
M.P. = 180-183°C
LC-MS [M+H] = 324
¹H-NMR (δ-ppm, CDCl₃) = 2.89 (s, 3H); 5.24 (s, 2H);
7.25-7.38 (m, 3H).

### EXAMPLE 3

### Preparation of 2-[(1-CH₃-3-CF₃-5-OCF₂H-pyrazol-4-yl)sulfinyl]-5-methyl-1,3,4-thiadiazole [Compound Nr.51]

### a) Preparation of (4-(bromomethyl)-5-(difluoromethoxy)-1-methyl-3-(trifluoromethyl)-1H-pyrazole [compound having general formula (IV)]

3.40 g (19.14 mmoles) of N-bromo-succinimide and a catalytic quantity of azaisobutyrronitrile were added at room temperature to a solution under nitrogen of 4 g (17.4 mmoles) of 5-(difluoromethoxy)-1,4-dimethyl-3-(trifluoromethyl-1*H*-pyrazole in 17 ml of carbon tetrachloride.

The mixture was left under stirring and reflux temperature, irradiated with a 300 W lamp for 5 hours.

After control in LC-MS, the mixture was poured into water and extracted three times with chloroform. The organic phases joined together were washed with water and a saturated solution of sodium chloride.

After anhydrification on sodium sulfate, filtration and evaporation of the solvent at reduced pressure, 4.78 g (15.5 mmoles) of the desired product were obtained, as a yellow oil. Yield 89.0% LC-MS [M+H] = 309.

### b) Preparation of 2-[5-difluoromethoxy-1-methyl-3-trifluoromethyl-1H-pyrazol-4-yl]methylthio-5-methyl-1,3,4-thiadiazole [thioether having general formula (II)]

2.10 ml (15.2 mmoles) of triethylamine were added dropwise at room temperature to a suspension under nitrogen of 2 g (15.2 mmoles) of 5-methyl-1,3,4-thiadiazole-2-thiol in 10 ml of chloroform; 4.70 g (15.2 mmoles) of (4-(bromomethyl)-5-(difluoromethoxy)-1-methyl-3-(trifluoromethyl)-1*H*-pyrazole, dissolved in 4 ml of chloroform were then added. Finally 3.15 ml (22.8 mmoles) of triethylamine were added dropwise.

The mixture was left under magnetic stirring at room temperature for a night.

After control in GC-MS and LC-MS, the mixture was diluted with water and the phases were then separated; the aqueous phase was re-extracted twice with dichloromethane. The organic phases joined together, were washed with water and a saturated solution of sodium chloride.

After anhydrification on sodium sulfate, filtration and evaporation of the solvent at reduced pressure, 4.57 g (68.0 mmoles) of the desired product were obtained, as a brown oil. Yield 83.5% LC-MS [M+H] = 361.

### c) Preparation of 2-[(1-CH₃-3-CF₃-5-OCF₂H-pyrazol-4-yl)sulfinyl]-5-methyl-1,3,4-thiadiazole [Compound N° 51]

3.08 g (13.75 mmoles) of 4-Cl-perbenzoic acid at 77% were added to 4.5 g (12.5 mmoles) of 2-({[5-(difluoromethoxy)-1-methyl-3-(trifluoromethyl)-1*H-*pyrazol-4-yl]methyl}thio-5-methyl-1,3,4-thiadiazole, dissolved in 50 ml of chloroform, maintaining a temperature of about 4-5°C with an ice bath; the mixture was then left under magnetic stirring at room temperature for a night.

After control in LC-MS, the mixture was diluted with water and the phases were then separated; the aqueous phase was re-extracted twice with dichloromethane. The organic phases joined together, were washed with an aqueous solution at 5% of NaHSO₃, a saturated solution of NaHCO₃, water and a saturated solution of NaCl.

After anhydrification on sodium sulfate, filtration and evaporation of the solvent at reduced pressure, 4.1 g (15.9 mmoles) of the desired product were obtained, as a yellow oil. The raw product thus obtained was crushed with ethyl ether, filtered and dried in the air, obtaining 3.7 g (9.87 mmoles) of the desired product as a white solid. Yield 79.0%.
LC-MS [M+H] = 377

### EXAMPLE 4

### Preparation of 2-[(1-CH₃-3-CF₃-5-OCF₂H-pyrazol-4-yl)sulfonyl]-5-methyl-1,3,4-thiadiazole [Compound Nr.52]

8.40 g (37.5 mmoles) of 4-chloro-perbenzoic acid at 77% were added to 4.5 g (12.5 mmoles) of 2-({[5-(difluoromethoxy)-1-methyl-3-(trifluoromethyl)-1*H-*pyrazol-4-yl]methyl}thio)-5-methyl-1,3,4-thiadiazole, dissolved in 50 ml of chloroform, maintaining a temperature of about 4-5°C with an ice bath; the mixture was then left under magnetic stirring at room temperature for a night.

After control in LC-MS, the mixture was diluted with water and the phases were then separated; the aqueous phase was re-extracted twice with dichloromethane. The organic phases joined together, were washed with an aqueous solution at 5% of NaHSO₃, a saturated solution of NaHCO₃, water and a saturated solution of NaCl.

After anhydrification on sodium sulfate, filtration and evaporation of the solvent at reduced pressure, 4.7 g (15.9 mmoles) of the desired product were obtained, as an orange oil. The raw product thus obtained was purified by flash chromatography, obtaining 3.47 g (8.87 mmoles) of the desired product as a white solid. Yield 71.0%.
LC-MS [M+H] = 393
¹H-NMR (δ-ppm, CDCl₃) = 2.91 (s, 3H); 3.86 (s, 3H); 4.75 (s, 2H); 6.81 (t, 1H).

### EXAMPLE 5

### Preparation of Compounds Nr.3-50, 53-124

Operating analogously to what is described in the previous examples, the compounds having general formula (I) were obtained, corresponding to compounds Nr.3-50 and 53-124, listed in Table 1.

Table 2 indicates the results of the GC-MS and/or LC-MS analyses carried out on the synthesized compounds.

**Table 2**

| **Nr.** | **GC-MS** | **LC-MS [M+H⁺]** |
|---|---|---|
| 1 | - | 308 |
| 2 | 323 | 324 |
| 3 | - | 275 |
| 4 | 290 | 291 |
| 5 | - | 291 |
| 6 | 306 | 307 |
| 7 | - | 308 |
| 8 | 323 | 324 |
| 9 | - | 409 |
| 10 | 424 | 425 |
| 11 | - | 318 |
| 12 | 333 | 334 |
| 13 | - | 351 |
| 14 | 366 | 367 |
| 15* | - | 284 |
| 16* | 299 | 300 |
| 17 | - | 253 |
| 18 | 268 | 269 |
| 19 | - | 337 |
| 20 | 352 | 353 |
| 21 | - | 423 |
| 22 | 438 | 439 |
| 23 | - | 267 |
| 24 | 282 | 283 |
| 25 | - | 299 |
| 26 | 314 | 315 |
| 27 | - | 307 |
| 28 | 322 | 323 |
| 29 | - | 329 |
| 30 | 344 | 345 |
| 31 | - | 393 |
| 32 | 408 | 409 |
| 33 | - | 349 |
| 34 | 364 | 365 |
| 35 | - | 339 |
| 36 | 354 | 355 |
| 37 | - | 301 |
| 38 | 316 | 317 |
| 39 | - | 286 |
| 40 | 301 | 302 |
| 41 | - | 274 |
| 42 | 305 | 306 |
| 43 | - | 274 |
| 44 | 305 | 306 |
| 45 | - | 240 |
| 46 | 271 | 272 |
| 47 | - | 240 |
| 48 | 271 | 272 |
| 49 | - | 321 |
| 50 | 336 | 337 |
| 51 | - | 377 |
| 52 | 392 | 393 |
| 53 | - | 293 |
| 54 | 308 | 309 |
| 55 | - | 257 |
| 56 | 272 | 273 |
| 57 | - | 391 |
| 58 | 406 | 407 |
| 59 | - | 405 |
| 60 | 420 | 421 |
| 61 | - | 363 |
| 62 | 378 | 379 |
| 63 | - | 391 |
| 64 | 406 | 407 |
| 65 | - | 419 |
| 66 | 434 | 435 |
| 67 | - | 417 |
| 68 | 432 | 433 |
| 69 | - | 335 |
| 70 | 350 | 351 |
| 71 | - | 321 |
| 72 | 336 | 337 |
| 73 | - | 293 |
| 74 | 308 | 309 |
| 75 | - | 321 |
| 76 | 336 | 337 |
| 77 | - | 349 |
| 78 | 364 | 365 |
| 79 | - | 335 |
| 80 | 350 | 351 |
| 81 | - | 347 |
| 82 | 362 | 363 |
| 83 | - | 321 |
| 84 | 336 | 337 |
| 85 | - | 333 |
| 86 | 348 | 349 |
| 87 | - | 375 |
| 88 | 390 | 391 |
| 89 | - | 389 |
| 90 | 404 | 405 |
| 91 | - | 347 |
| 92 | 362 | 363 |
| 93 | - | 405 |
| 94 | 420 | 421 |
| 95 | - | 391 |
| 96 | 406 | 407 |
| 97 | - | 403 |
| 98 | 418 | 419 |
| 99 | - | 445 |
| 100 | 460 | 461 |
| 101 | - | 459 |
| 102 | 474 | 475 |
| 103 | - | 417 |
| 104 | 432 | 433 |
| 105 | - | 425 |
| 106 | 440 | 441 |
| 107 | - | 425 |
| 108 | 440 | 441 |
| 109 | - | 425 |
| 110 | 440 | 441 |
| 111 | - | 261 |
| 112 | 276 | 277 |
| 113 | - | 328 |
| 114 | 343 | 344 |
| 115 | - | 345 |
| 116 | 360 | 361 |
| 117 | - | 335 |
| 118 | 350 | 351 |
| 119 | - | 379 |
| 120 | 394 | 395 |
| 121 | - | 423 |
| 122 | 438 | 439 |
| 123 | - | 429 |
| 124 | 444 | 445 |

| | | |
|---|---|---|
| * compounds not according to the invention. | | |

### EXAMPLE 6

### Determination of the herbicidal activity and phytotoxicity in pre-emergence.

The herbicidal activity in pre-emergence of the compounds of the invention was evaluated according to the following operating procedures.

The plant species of interest (weeds or crops) were seeded in vases having an upper diameter of 10 cm, a height of 10 cm and containing sandy earth. 10 vases were used for each plant species.

Water was added to each vase in a suitable quantity for the germination of the seeds. The vases were then divided into two groups, each containing 5 vases for each weed or crop.

One day after seeding, the first group of vases was treated with a dispersion having the following composition:

| | |
|---|---|
| - Compound having general formula (I) | 50 mg |
| - Water | 56 ml |
| - Acetone | 14 ml |
| - Tween 20 | 0.5% |

Said dispersion was prepared by adding a compound of the present invention at the desired concentration to a hydroacetonic solution containing acetone at 20% by volume and Tween 20 at 0.5% by weight, with respect to the total weight of the dispersion.

The desired concentration of a compound of the present invention corresponds to the concentration necessary for allowing the application of an effective dose of said compound equal to 250 g/ha to the plant species of interest.

The second group was only treated with a hydroacetonic solution containing acetone at 20% by volume and Tween 20 at 0.5% by weight, and was used as a comparison (control).

All the vases were kept under observation in a conditioned environment under the following environmental conditions:
- temperature: 24°C;
- relative humidity: 60%;
- photo-period: 16 hours;
- light intensity: 12,000 lux.

Every two days the vases were uniformly watered to ensure a sufficient humidity degree for a good growth of the plants.

Fifteen days after treatment, the herbicidal activity was evaluated on the basis of the following scale of values which refers to the percentage of damage revealed on the treated plants with respect to the non-treated plants (control).

| | |
|---|---|
| - 0 | = 0 - 10 % of damage; |
| - 1 | = 11 - 30 % of damage; |
| - 2 | = 31 - 50 % of damage; |
| - 3 | = 51 - 70 % of damage; |
| - 4 | = 71 - 90 % of damage; |
| - 5 | = 91 % of damage - death of the plant. |

Table 3 shows the results obtained by treating the plant species indicated below with compounds **Nr.1**, **Nr.9**, **Nr.51 and Nr. 121**, compared with a dispersion containing the compound CR1 described in DE 2533604, instead of the compound having general formula (I):

**CR1** = 2-(2,6-dichlorophenylsulfonyl)-5-(trifluoromethyl)-1,3,4-thiadiazole

**Table 3: Herbicidal activity in pre-emergence at a dose of 250 g/ha**

| **Weeds** | **Compounds** | | | | |
|---|---|---|---|---|---|
| | **Nr.1** | **Nr.9** | **Nr.51** | **Nr.121** | **CR1** |
| Echinochloa crusgalli | 4 | 5 | 5 | 5 | 0 |
| Digitaria sanguinalis | 5 | 5 | 5 | 5 | 0 |
| Setaria viridis | 4 | 5 | 5 | 5 | 0 |
| Eleusine indica | 5 | 5 | 5 | 5 | 0 |
| Lolium rigidum | 4 | 5 | 4 | 5 | 0 |
| Poa Annua | 5 | 5 | 5 | 5 | 0 |
| Apera spica-venti | 5 | 5 | 5 | 5 | 0 |
| Alopecurus myosuroides | 4 | 5 | 5 | 5 | 0 |

### EXAMPLE 7

### Determination of the herbicidal activity and phytotoxicity in post-emergence.

The herbicidal activity in post-emergence of the compounds of the invention was evaluated according to the following operating procedures.

The plant species of interest (weeds or crops) were seeded in vases having an upper diameter of 10 cm, a height of 10 cm and containing sandy earth. 10 vases were used for each plant species.

Water was added to each vase in a suitable quantity for the germination of the seeds. The vases were then divided into two groups, each containing 5 vases for each weed or crop.

Fifteen days after seeding (ten in the case of wheat), i.e. when the weeds and crops, depending on the species, had a height of 10-15 cm, the first group of vases was treated with the same hydroacetonic dispersion containing acetone at 20% by volume, the compound being evaluated at the desired concentration and Tween 20 at 0.5% by weight, indicated in Example 6.

The second group was only treated with a hydroacetonic solution containing acetone at 20% by volume and Tween 20 at 0.5% by weight, and was used as a comparison (control).

All the vases were kept under observation in a conditioned environment under the following environmental conditions:
- temperature: 24°C;
- relative humidity: 60%;
- photo-period: 16 hours;
- light intensity: 12,000 lux.

Every two days the vases were uniformly watered to ensure a sufficient humidity degree for a good growth of the plants.

Fifteen days after treatment, the herbicidal activity was evaluated on the basis of the following scale of values which refers to the percentage of damage revealed on the treated plants with respect to the non-treated plants (control).

| | | |
|---|---|---|
| - 0 | = | 0 - 10 % of damage; |
| - 1 | = | 11 - 30 % of damage; |
| - 2 | = | 31 - 50 % of damage; |
| - 3 | = | 51 - 70 % of damage; |
| - 4 | = | 71 - 90 % of damage; |
| - 5 | = | 91 % of damage - death of the plant. |

Table 4 shows the results obtained by treating the vegetable species indicated below with compounds **Nr.1**, **Nr.9**, **Nr.51 and Nr.121** compared with a hydroacetonic dispersion containing the compound CR1 described in DE 2533604:

**Table 4: Herbicidal activity in post-emergence at a dose of 250 g/ha**

| **Weeds** | **Compounds** | | | | |
|---|---|---|---|---|---|
| | **Nr.1** | **Nr.9** | **Nr.51** | **Nr.121** | **CR1** |
| Echinochloa crusgalli | 4 | 5 | 5 | 5 | 0 |
| Digitaria sanguinalis | 5 | 5 | 5 | 5 | 0 |
| Setaria viridis | 4 | 4 | 4 | 4 | 0 |
| Eleusine indica | 5 | 5 | 5 | 5 | 0 |
| Lolium rigidum | 4 | 4 | 4 | 4 | 0 |
| Poa Annua | 5 | 5 | 5 | 5 | 0 |
| Apera spica-venti | 5 | 5 | 5 | 5 | 0 |
| Alopecurus myosuroides | 4 | 5 | 4 | 4 | 0 |

Compounds Nr.2-8 and 10-124 tested under the same conditions of pre- and post-emergence showed a herbicidal activity of at least 50% with respect to the weeds indicated in Tables 3 and 4.

## Claims

1. Thiadiazoles having general formula (I), in racemic form, isomerically pure, or mixtures thereof: 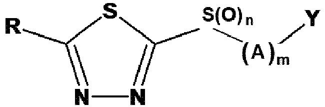 wherein R, R₁, R₂, Y, n and m have the meanings specified hereunder:
| **N°** | **R** | **R₁** | **R₂** | **Y** | **n** | **m** |
|---|---|---|---|---|---|---|
| 1 | CH₃ | H | H | 2,6-diCl-phenyl | 1 | 1 |
| 2 | CH₃ | H | H | 2,6-diCl-phenyl | 2 | 1 |
| 3 | CH₃ | H | H | 2,6-diF-phenyl | 1 | 1 |
| 4 | CH₃ | H | H | 2,6-diF-phenyl | 2 | 1 |
| 5 | CH₃ | H | H | 2-Cl-6-F-phenyl | 1 | 1 |
| 6 | CH₃ | H | H | 2-Cl-6-F-phenyl | 2 | 1 |
| 7 | CH₃ | H | H | 2,4-diCl-phenyl | 1 | 1 |
| 8 | CH₃ | H | H | 2,4-diCl-phenyl | 2 | 1 |
| 9 | CH₃ | H | H | 1-CH₃-3-CF₃-5-OCH₂CF₃-pyrazol-4-yl | 1 | 1 |
| 10 | CH₃ | H | H | 1-CH₃-3-CF₃-5-OCH₂CF₃-pyrazol-4-yl | 2 | 1 |
| 11 | CH₃ | H | H | 2-Cl-4,5-methyl-endioxy-phenyl | 1 | 1 |
| 12 | CH₃ | H | H | 2-Cl-4,5-methyl-endioxy-phenyl | 2 | 1 |
| 13 | CH₃ | H | H | 2,5-diCl-4-OEt-phenyl | 1 | 1 |
| 14 | CH₃ | H | H | 2,5-diCl-4-OEt-phenyl | 2 | 1 |
| 17 | CH₃ | H | H | 2-CH₃-phenyl | 1 | 1 |
| 18 | CH₃ | H | H | 2-CH₃-phenyl | 2 | 1 |
| 19 | CH₃ | H | H | 2,5-diCl-4-OMe-phenyl | 1 | 1 |
| 20 | CH₃ | H | H | 2,5-diCl-4-OCF₂CF₂H -phenyl | 2 | 1 |
| 21 | CH₃ | H | H | 2,5-diCl-4-OCF₂CF₂H -phenyl | 1 | 1 |
| 22 | CH₃ | H | H | 2,5-diCl-4-OMe-phenyl | 2 | 1 |
| 23 | CH₃ | H | H | 2,5-diCH₃-phenyl | 1 | 1 |
| 24 | CH₃ | H | H | 2,5-diCH₃-phenyl | 2 | 1 |
| 25 | CH₃ | H | H | 2,6-diOCH₃-phenyl | 1 | 1 |
| 26 | CH₃ | H | H | 2,6-diOCH₃-phenyl | 2 | 1 |
| 27 | CH₃ | H | H | 3-CF₃-phenyl | 1 | 1 |
| 28 | CH₃ | H | H | 2-CF₃-phenyl | 2 | 1 |
| 29 | CH₃ | H | H | 2,3,4,5,6-pentaF-phenyl | 1 | 1 |
| 30 | CH₃ | H | H | 2,3,4,5,6-pentaF-phenyl | 2 | 1 |
| 31 | CH₃ | H | H | 2-SO₂Me-5-COCH₃-6-Cl-phenyl | 1 | 1 |
| 32 | CH₃ | H | H | 2-SO₂Me-5-COCH₃-6-Cl-phenyl | 2 | 1 |
| 33 | CH₃ | H | H | 2,6-diCl-5-COCH₃-phenyl | 1 | 1 |
| 34 | CH₃ | H | H | 2,6-diCl-5-COCH₃-phenyl | 2 | 1 |
| 35 | CH₃ | H | H | 2-CH₃-5-Br-thiazol-2-yl | 1 | 1 |
| 36 | CH₃ | H | H | 2-CH₃-5-Br-thiazol-2-yl | 2 | 1 |
| 37 | CH₃ | H | H | 3-COOEt-furan-2-yl | 1 | 1 |
| 38 | CH₃ | H | H | 3-COOEt-furan-2-yl | 2 | 1 |
| 39 | CH₃ | H | H | 3-*t*Bu-isoxazol-2-yl | 1 | 1 |
| 40 | CH₃ | H | H | 3-*t*Bu-isoxazol-2-yl | 2 | 1 |
| 41 | CH₃ | H | H | 6-Cl-pyiridin-3-yl | 1 | 1 |
| 42 | CH₃ | H | H | 6-Cl-pyridin-3-yl-N-oxide | 2 | 1 |
| 43 | CH₃ | H | H | 6-Cl-pyridin-2-yl | 1 | 1 |
| 44 | CH₃ | H | H | 6-Cl-pyridin-2-yl-N-oxide | 2 | 1 |
| 45 | CH₃ | H | H | pyridin-3-yl | 1 | 1 |
| 46 | CH₃ | H | H | pyridin-3-yl-N-oxide | 2 | 1 |
| 47 | CH₃ | H | H | pyridin-2-yl | 1 | 1 |
| 48 | CH₃ | H | H | pyridin-2-yl-N-oxide | 2 | 1 |
| 49 | CH₃ | H | CH₃ | 2,4-diCl-phenyl | 1 | 1 |
| 50 | CH₃ | H | CH₃ | 2,4-diCl-phenyl | 2 | 1 |
| 51 | CH₃ | H | H | 1-CH₃-3-CF₃-5-OCF₂H-pyrazol-4-yl | 1 | 1 |
| 52 | CH₃ | H | H | 1-CH₃-3-CF₃-5-OCF₂H-pyrazol-4-yl | 2 | 1 |
| 53 | CH₃ | H | H | 1-CH₃-3-CF₂H-pyrazol-4-yl | 1 | 1 |
| 54 | CH₃ | H | H | 1-CH₃-3-CF₂H-pyrazol-4-yl | 2 | 1 |
| 55 | CH₃ | H | H | 4-F-phenyl | 1 | 1 |
| 56 | CH₃ | H | H | 4-F-phenyl | 2 | 1 |
| 57 | CH₃ | H | H | 1-CH₃-3-CF₃-5-OCF₂H-pyrazol-4-yl | 1 | 2 |
| 58 | CH₃ | H | H | 1-CH₃-3-CF₃-5-OCF₂H-pyrazol-4-yl | 2 | 2 |
| 59 | CH₃ | H | H | 1-CH₃-3-CF₃-5-OCF₂H-pyrazol-4-yl | 1 | 3 |
| 60 | CH₃ | H | H | 1-CH₃-3-CF₃-5-OCF₂H-pyrazol-4-yl | 2 | 3 |
| 63 | CH₃ | H | CH₃ | 1-CH₃-3-CF₃-5-OCF₂H-pyrazol-4-yl | 1 | 1 |
| 64 | CH₃ | H | CH₃ | 1-CH₃-3-CF₃-5-OCF₂H-pyrazol-4-yl | 2 | 1 |
| 65 | CH₃ | H | CH₃ | 1-CH₃-3-CF₃-5-OCF₂H-pyrazol-4-yl | 1 | 2 |
| 66 | CH₃ | H | CH₃ | 1-CH₃-3-CF₃-5-OCF₂H-pyrazol-4-yl | 2 | 2 |
| 67 | CH₃ | H | C₃H₅ | 1-CH₃-3-CF₃-5-OCF₂H-pyrazol-4-yl | 1 | 1 |
| 68 | CH₃ | H | C₃H₅ | 1-CH₃-3-CF₃-5-OCF₂H-pyrazol-4-yl | 2 | 1 |
| 69 | CH₃ | H | H | 2,6-diCl-phenyl | 1 | 3 |
| 70 | CH₃ | H | H | 2,6-diCl-phenyl | 2 | 3 |
| 71 | CH₃ | H | H | 2,6-diCl-phenyl | 1 | 2 |
| 72 | CH₃ | H | H | 2,6-diCl-phenyl | 2 | 2 |
| 75 | CH₃ | H | CH₃ | 2,6-diCl-phenyl | 1 | 1 |
| 76 | CH₃ | H | CH₃ | 2,6-diCl-phenyl | 2 | 1 |
| 77 | CH₃ | H | CH₃ | 2,6-diCl-phenyl | 1 | 2 |
| 78 | CH₃ | H | CH₃ | 2,6-diCl-phenyl | 2 | 2 |
| 79 | CH₃ | CH₃ | CH₃ | 2,6-diCl-phenyl | 1 | 1 |
| 80 | CH₃ | CH₃ | CH₃ | 2,6-diCl-phenyl | 2 | 1 |
| 81 | CH₃ | H | C₃H₅ | 2,6-diCl-phenyl | 1 | 1 |
| 82 | CH₃ | H | C₃H₅ | 2,6-diCl-phenyl | 2 | 1 |
| 83 | C₂H₅ | H | H | 2,6-diCl-phenyl | 1 | 1 |
| 84 | C₂H₅ | H | H | 2,6-diCl-phenyl | 2 | 1 |
| 85 | C₃H₅ | H | H | 2,6-diCl-phenyl | 1 | 1 |
| 86 | C₃H₅ | H | H | 2,6-diCl-phenyl | 2 | 1 |
| 87 | C₆H₁₁ | H | H | 2,6-diCl-phenyl | 1 | 1 |
| 88 | C₆H₁₁ | H | H | 2,6-diCl-phenyl | 2 | 1 |
| 89 | C₆H₁₁CH₂ | H | H | 2,6-diCl-phenyl | 1 | 1 |
| 90 | C₆H₁₁CH₂ | H | H | 2,6-diCl-phenyl | 2 | 1 |
| 91 | C₃H₅CH₂ | H | H | 2,6-diCl-phenyl | 1 | 1 |
| 92 | C₃H₅CH₂ | H | H | 2,6-diCl-phenyl | 2 | 1 |
| 93 | CH₃ | CH₃ | CH₃ | 1-CH₃-3-CF₃-5-OCF₂H-pyrazol-4-yl | 1 | 1 |
| 94 | CH₃ | CH₃ | CH₃ | 1-CH₃-3-CF₃-5-OCF₂H-pyrazol-4-yl | 2 | 1 |
| 95 | C₂H₅ | H | H | 1-CH₃-3-CF₃-5-OCF₂H-pyrazol-4-yl | 1 | 1 |
| 96 | C₂H₅ | H | H | 1-CH₃-3-CF₃-5-OCF₂H-pyrazol-4-yl | 2 | 1 |
| 97 | C₃H₅ | H | H | 1-CH₃-3-CF₃-5-OCF₂H-pyrazol-4-yl | 1 | 1 |
| 98 | C₃H₅ | H | H | 1-CH₃-3-CF₃-5-OCF₂H-pyrazol-4-yl | 2 | 1 |
| 99 | C₆H₁₁ | H | H | 1-CH₃-3-CF₃-5-OCF₂H-pyrazol-4-yl | 1 | 1 |
| 100 | C₆H₁₁ | H | H | 1-CH₃-3-CF₃-5-OCF₂H-pyrazol-4-yl | 2 | 1 |
| 101 | C₆H₁₁CH₂ | H | H | 1-CH₃-3-CF₃-5-OCF₂H-pyrazol-4-yl | 1 | 1 |
| 102 | C₆H₁₁CH₂ | H | H | 1-CH₃-3-CF₃-5-OCF₂H-pyrazol-4-yl | 2 | 1 |
| 103 | C₃H₅CH₂ | H | H | 1-CH₃-3-CF₃-5-OCF₂H-pyrazol-4-yl | 1 | 1 |
| 104 | C₃H₅CH₂ | H | H | 1-CH₃-3-CF₃-5-OCF₂H-pyrazol-4-yl | 2 | 1 |
| 105 | CH₃ | H | H | 2-CF₃-4-F-phenyl | 1 | 1 |
| 106 | CH₃ | H | H | 2-CF₃-4-F-phenyl | 2 | 1 |
| 107 | CH₃ | H | H | 2-CF₃-5-F-phenyl | 1 | 1 |
| 108 | CH₃ | H | H | 2-CF₃-5-F-phenyl | 2 | 1 |
| 109 | CH₃ | H | H | 2-CF₃-6-F-phenyl | 1 | 1 |
| 110 | CH₃ | H | H | 2-CF₃-6-F-phenyl | 2 | 1 |
| 111 | CH₃ | H | H | 5-CH₃-1, 3, 4-thiadiazol-4-yl | 1 | 1 |
| 112 | CH₃ | H | H | 5-CH₃-1, 3, 4-thiadiazol-4-yl | 2 | 1 |
| 113 | CH₃ | H | H | 2-CH₃-4-CF₃-1, 3-thiazol-5-yl | 1 | 1 |
| 114 | CH₃ | H | H | 2-CH₃-4-CF₃-1, 3-thiazol-5-yl | 2 | 1 |
| 115 | CH₃ | H | H | 1-CH₃-3-CF₃-5-Cl-pyrazol-4-yl | 1 | 1 |
| 116 | CH₃ | H | H | 1-CH₃-3-CF₃-5-Cl-pyrazol-4-yl | 2 | 1 |
| 117 | *i*C₃H₇ | H | H | 2,6-diCl-phenyl | 1 | 1 |
| 118 | *i*C₃H₇ | H | H | 2,6-diCl-phenyl | 2 | 1 |
| 119 | *i*C₃H₇ | H | H | 2,5-diCl-4-OEt-phenyl | 1 | 1 |
| 120 | *i*C₃H₇ | H | H | 2,5-diCl-4-OEt-phenyl | 2 | 1 |
| 121 | C₂H₅ | H | H | 1-CH₃-3-CF₃-5-OCH₂CF₃-pyrazol-4-yl | 1 | 1 |
| 122 | C₂H₅ | H | H | 1-CH₃-3-CF₃-5-OCH₂CF₃-pyrazol-4-yl | 2 | 1 |
| 123 | Cl | H | H | 1-CH₃-3-CF₃-5-OCH₂CF₃-pyrazol-4-yl | 1 | 1 |
| 124 | Cl | H | H | 1-CH₃-3-CF₃-5-OCH₂CF₃-pyrazol-4-yl | 2 | 1 |

2. The thiadiazoles according to claim 1, selected from compounds having general formula (I), wherein:
| **N°** | **R** | **R₁** | **R₂** | **Y** | **n** | **m** |
|---|---|---|---|---|---|---|
| 1 | CH₃ | H | H | 2,6-diCl-phenyl | 1 | 1 |
| 51 | CH₃ | H | H | 1-CH₃-3-CF₃-5-OCF₂H-pyrazol-4-yl | 1 | 1 |
| 9 | CH₃ | H | H | 1-CH₃-3-CF₃-5-OCH₂CF₃-pyrazol-4-yl | 1 | 1 |
| 83 | C₂H₅ | H | H | 2,6-diCl-phenyl | 1 | 1 |
| 95 | C₂H₅ | H | H | 1-CH₃-3-CF₃-5-OCF₂H-pyrazol-4-yl | 1 | 1 |
| 121 | C₂H₅ | H | H | 1-CH₃-3-CF₃-5-OCH₂CF₃-pyrazol-4-yl | 1 | 1 |

3. Herbicidal compositions comprising at least one compound having formula (I) according to any of the previous claims, at least one solvent and/or diluent, possibly one or more agronomically acceptable excipients selected from: surfactant, dispersant, stabilizer and mixtures thereof.

4. The herbicidal compositions according to the previous claim also comprising at least one active ingredient compatible with the compounds having general formula (I) selected from: herbicides different from those having general formula (I), fungicides, insecticides, acaricides, fertilizers and mixtures thereof.

5. The herbicidal compositions according to one or more of claims 3-4, wherein said compound having general formula (I) has a concentration ranging from 1% to 90% by weight with respect to the total weight of the composition.

6. Use of thiadiazoles having general formula (I) 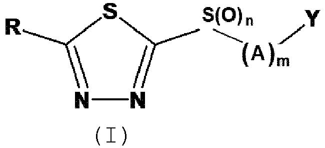 wherein:
- R represents a halogen atom, a C₁-C₄ alkyl, a C₃-C₆ cycloalkyl or a C₄-C₇ cycloalkylalkyl;
- A represents a group CR₁R₂, wherein R₁ and R₂, the same or different, represent a hydrogen atom, a C₁-C₄ alkyl, a C₃-C₆ cycloalkyl;
- Y represents a phenyl, a naphthyl, or an aromatic heterocyclic group, these groups being optionally substituted with at least one substituent selected from: halogen atoms, C₁-C₄ alkyl, C₁-C₄ haloalkyl, C₁-C₄ alkoxy, C₁-C₄ haloalkoxy, C₃-C₆ cycloalkoxy, C₄-C₇ cycloalkylalkoxy, phenoxy, C₁-C₄ alkylthio, C₁-C₄ haloalkylthio, C₁-C₄ alkylsulfinyl, C₁-C₄ alkylsulfonyl, C₁-C₄ alkylamino, C₂-C₈ dialkylamino, cyano, C₂-C₅ alkoxycarbonyl, benzyloxycarbonyls, phenoxycarbonyls, or two substituents together represent a C₁-C₄ alkylenedioxy group; in the case of the aromatic heterocyclic group, said at least one substituent also being selected from nitro and carboxyl;
- n represents an integer from 1 to 2;
- m represents an integer from 1 to 6. as herbicides for the control of at least one weed in an agricultural crop.

7. Use according to claim 6 for the control of at least one weed in pre-emergence or post-emergence.

8. Use according to claim 6 or 7, wherein said weed is selected from: Abutilon theofrasti, Alisma plantago, Amaranthus spp., Amni maius, Capsella bursa pastoris, Chenopodium album, Convolvulus sepium, Galium aparine, Geranium dissectum, Heteranthera spp., Ipomea spp., Matricaria spp., Papaver rhoaes, Phaseolus aureus, Polygonum persicaria, Portulaca oleracea, Setaria viridis, Sida spinosa, Sinapsis arvensis, Solanum nigrum, Stellaria media, Veronica spp., Viola spp., Xanthium spp., Alopecurus myosuroides, Anisanta spp., Apera spica-venti, Avena spp., Cyperus spp., Digitaria sanguinalis, Eleusine spp. Echinochloa spp., Eleocharis avicularis, Lolium spp., Panicum spp., Poa spp., Scirpus spp., Sorghum spp..

9. Use according to one or more of claims 6-8, wherein said agricultural crop is selected from wheat (Triticum sp.), barley (Hordeum vulgare), corn (Zea mays), soya (Glycine max).

10. A method for controlling at least one weed in an agricultural crop which comprises applying to the agricultural crop at least one effective dose of at least one compound having general formula (I) 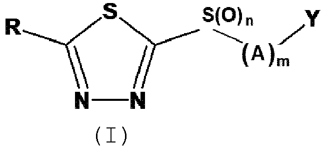 wherein:
- R represents a halogen atom, a C₁-C₄ alkyl, a C₃-C₆ cycloalkyl or a C₄-C₇ cycloalkylalkyl;
- A represents a group CR₁R₂, wherein R₁ and R₂, the same or different, represent a hydrogen atom, a C₁-C₄ alkyl, a C₃-C₆ cycloalkyl;
- Y represents a phenyl, a naphthyl, or an aromatic heterocyclic group, these groups being optionally substituted with at least one substituent selected from: halogen atoms, C₁-C₄ alkyl, C₁-C₄ haloalkyl, C₁-C₄ alkoxy, C₁-C₄ haloalkoxy, C₃-C₆ cycloalkoxy, C₄-C₇ cycloalkylalkoxy, phenoxy, C₁-C₄ alkylthio, C₁-C₄ haloalkylthio, C₁-C₄ alkylsulfinyl, C₁-C₄ alkylsulfonyl, C₁-C₄ alkylamino, C₂-C₈ dialkylamino, cyano, C₂-C₅ alkoxycarbonyl, benzyloxycarbonyls, phenoxycarbonyls, or two substituents together represent a C₁-C₄ alkylenedioxy group; in the case of the aromatic heterocyclic group, said at least one substituent also being selected from nitro and carboxyl;
- n represents an integer from 1 to 2;
- m represents an integer from 1 to 6..

11. The method according to the previous claim, wherein said compound having general formula (I) or said herbicidal composition is applied to the agricultural crop in a dosage of said compound having general formula (I) within the range of 1-1,000 g/ha.

12. A process for preparing thiadiazoles having general formula (I) comprising the phase of oxidizing a compound having general formula (II) with at least one oxidizing agent in at least one solvent, according to the scheme 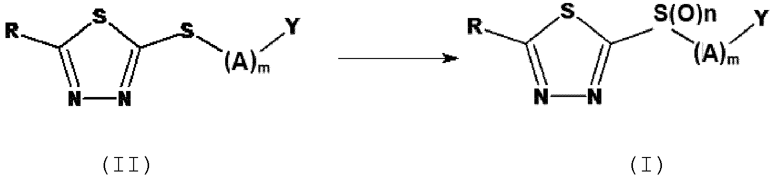 wherein R, R₁, R₂, Y, n and m have the meanings defined in claim 1.

13. The process according to claim 12, wherein said oxidizing agent is selected from an organic peroxide, such as 4-chloro-perbenzoic acid, peracetic acid or an inorganic peroxide, such as hydrogen peroxide, potassium permanganate, sodium periodate.

## Patentansprüche

1. Thiadiazole mit der allgemeinen Formel (I) in racemischer Form, isometrisch rein oder Gemische davon: 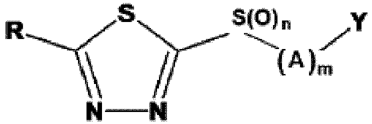 wobei R, R₁, R₂, Y, n und m die nachfolgend angegebenen Bedeutungen aufweisen:
| **Nr.** | **R** | **R₁** | **R₂** | **Y** | **n** | **m** |
|---|---|---|---|---|---|---|
| 1 | CH₃ | H | H | 2,6-DiCl-phenyl | 1 | 1 |
| 2 | CH₃ | H | H | 2,6-DiCl-phenyl | 2 | 1 |
| 3 | CH₃ | H | H | 2,6-DiF-phenyl | 1 | 1 |
| 4 | CH₃ | H | H | 2,6-DiF-phenyl | 2 | 1 |
| 5 | CH₃ | H | H | 2-Cl-6-F-phenyl | 1 | 1 |
| 6 | CH₃ | H | H | 2-Cl-6-F-phenyl | 2 | 1 |
| 7 | CH₃ | H | H | 2,4-DiCl-phenyl | 1 | 1 |
| 8 | CH₃ | H | H | 2,4-DiCl-phenyl | 2 | 1 |
| 9 | CH₃ | H | H | 1-CH₃-3-CF₃-5-OCH₂CF₃-pyrazol-4-yl | 1 | 1 |
| 10 | CH₃ | H | H | 1-CH₃-3-CF₃-5-OCH₂CF₃-pyrazol-4-yl | 2 | 1 |
| 11 | CH₃ | H | H | 2-Cl-4,5-methylendioxyphenyl | 1 | 1 |
| 12 | CH₃ | H | H | 2-Cl-4,5-methylendioxyphenyl | 2 | 1 |
| 13 | CH₃ | H | H | 2,5-DiCl-4-OEt-phenyl | 1 | 1 |
| 14 | CH₃ | H | H | 2,5-DiCl-4-OEt-phenyl | 2 | 1 |
| 17 | CH₃ | H | H | 2-CH₃-phenyl | 1 | 1 |
| 18 | CH₃ | H | H | 2-CH₃-phenyl | 2 | 1 |
| 19 | CH₃ | H | H | 2,5-DiCl-4-OMe-phenyl | 1 | 1 |
| 20 | CH₃ | H | H | 2,5-DiCl-4-OCF₂CF₂H-phenyl | 2 | 1 |
| 21 | CH₃ | H | H | 2,5-DiCl-4-OCF₂CF₂H-phenyl | 1 | 1 |
| 22 | CH₃ | H | H | 2,5-DiCl-4-OMe-phenyl | 2 | 1 |
| 23 | CH₃ | H | H | 2,5-DiCH₃-phenyl | 1 | 1 |
| 24 | CH₃ | H | H | 2,5-DiCH₃-phenyl | 2 | 1 |
| 25 | CH₃ | H | H | 2,6-DiOCH₃-phenyl | 1 | 1 |
| 26 | CH₃ | H | H | 2,6-DiOCH₃-phenyl | 2 | 1 |
| 27 | CH₃ | H | H | 3-CF₃-phenyl | 1 | 1 |
| 28 | CH₃ | H | H | 2-CF₃-phenyl | 2 | 1 |
| 29 | CH₃ | H | H | 2,3,4,5,6-PentaF-phenyl | 1 | 1 |
| 30 | CH₃ | H | H | 2,3,4,5,6-PentaF-phenyl | 2 | 1 |
| 31 | CH₃ | H | H | 2-SO₂Me-5-COCH₃-6-Cl-phenyl | 1 | 1 |
| 32 | CH₃ | H | H | 2-SO₂Me-5-COCH₃-6-Cl-phenyl | 2 | 1 |
| 33 | CH₃ | H | H | 2,6-DiCl-5-COCH₃-phenyl | 1 | 1 |
| 34 | CH₃ | H | H | 2,6-DiCl-5-COCH₃-phenyl | 2 | 1 |
| 35 | CH₃ | H | H | 2-CH₃-5-Br-thiazol-2-yl | 1 | 1 |
| 36 | CH₃ | H | H | 2-CH₃-5-Br-thiazol-2-yl | 2 | 1 |
| 37 | CH₃ | H | H | 3-COOEt-furan-2-yl | 1 | 1 |
| 38 | CH₃ | H | H | 3-COOEt-furan-2-yl | 2 | 1 |
| 39 | CH₃ | H | H | 3-*t*Bu-isoxazol-2-yl | 1 | 1 |
| 40 | CH₃ | H | H | 3-*t*Bu-isoxazol-2-yl | 2 | 1 |
| 41 | CH₃ | H | H | 6-Cl-pyiridin-3-yl | 1 | 1 |
| 42 | CH₃ | H | H | 6-Cl-pyridin-3-yl-N-oxid | 2 | 1 |
| 43 | CH₃ | H | H | 6-Cl-pyridin-2-yl | 1 | 1 |
| 44 | CH₃ | H | H | 6-Cl-pyridin-2-yl-N-oxid | 2 | 1 |
| 45 | CH₃ | H | H | Pyridin-3-yl | 1 | 1 |
| 46 | CH₃ | H | H | Pyridin-3-yl-N-oxid | 2 | 1 |
| 47 | CH₃ | H | H | Pyridin-2-yl | 1 | 1 |
| 48 | CH₃ | H | H | Pyridin-2-yl-N-oxid | 2 | 1 |
| 49 | CH₃ | H | CH₃ | 2,4-DiCl-phenyl | 1 | 1 |
| 50 | CH₃ | H | CH₃ | 2,4-DiCl-phenyl | 2 | 1 |
| 51 | CH₃ | H | H | 1-CH₃-3-CF₃-5-OCF₂H-pyrazol-4-yl | 1 | 1 |
| 52 | CH₃ | H | H | 1-CH₃-3-CF₃-5-OCF₂H-pyrazol-4-yl | 2 | 1 |
| 53 | CH₃ | H | H | 1-CH₃-3-CF₂H-pyrazol-4-yl | 1 | 1 |
| 54 | CH₃ | H | H | 1-CH₃-3-CF₂H-pyrazol-4-yl | 2 | 1 |
| 55 | CH₃ | H | H | 4-F-phenyl | 1 | 1 |
| 56 | CH₃ | H | H | 4-F-phenyl | 2 | 1 |
| 57 | CH₃ | H | H | 1-CH₃-3-CF₃-5-OCF₂H-pyrazol-4-yl | 1 | 2 |
| 58 | CH₃ | H | H | 1-CH₃-3-CF₃-5-OCF₂H-pyrazol-4-yl | 2 | 2 |
| 59 | CH₃ | H | H | 1-CH₃-3-CF₃-5-OCF₂H-pyrazol-4-yl | 1 | 3 |
| 60 | CH₃ | H | H | 1-CH₃-3-CF₃-5-OCF₂H-pyrazol-4-yl | 2 | 3 |
| 63 | CH₃ | H | CH₃ | 1-CH₃-3-CF₃-5-OCF₂H-pyrazol-4-yl | 1 | 1 |
| 64 | CH₃ | H | CH₃ | 1-CH₃-3-CF₃-5-OCF₂H-pyrazol-4-yl | 2 | 1 |
| 65 | CH₃ | H | CH₃ | 1-CH₃-3-CF₃-5-OCF₂H-pyrazol-4-yl | 1 | 2 |
| 66 | CH₃ | H | CH₃ | 1-CH₃-3-CF₃-5-OCF₂H-pyrazol-4-yl | 2 | 2 |
| 67 | CH₃ | H | C₃H₅ | 1-CH₃-3-CF₃-5-OCF₂H-pyrazol-4-yl | 1 | 1 |
| 68 | CH₃ | H | C₃H₅ | 1-CH₃-3-CF₃-5-OCF₂H-pyrazol-4-yl | 2 | 1 |
| 69 | CH₃ | H | H | 2,6-DiCl-phenyl | 1 | 3 |
| 70 | CH₃ | H | H | 2,6-DiCl-phenyl | 2 | 3 |
| 71 | CH₃ | H | H | 2,6-DiCl-phenyl | 1 | 2 |
| 72 | CH₃ | H | H | 2,6-DiCl-phenyl | 2 | 2 |
| 75 | CH₃ | H | CH₃ | 2,6-DiCl-phenyl | 1 | 1 |
| 76 | CH₃ | H | CH₃ | 2,6-DiCl-phenyl | 2 | 1 |
| 77 | CH₃ | H | CH₃ | 2,6-DiCl-phenyl | 1 | 2 |
| 78 | CH₃ | H | CH₃ | 2,6-DiCl-phenyl | 2 | 2 |
| 79 | CH₃ | CH₃ | CH₃ | 2,6-DiCl-phenyl | 1 | 1 |
| 80 | CH₃ | CH₃ | CH₃ | 2,6-DiCl-phenyl | 2 | 1 |
| 81 | CH₃ | H | C₃H₅ | 2,6-DiCl-phenyl | 1 | 1 |
| 82 | CH₃ | H | C₃H₅ | 2,6-DiCl-phenyl | 2 | 1 |
| 83 | C₂H₅ | H | H | 2,6-DiCl-phenyl | 1 | 1 |
| 84 | C₂H₅ | H | H | 2,6-DiCl-phenyl | 2 | 1 |
| 85 | C₃H₅ | H | H | 2,6-DiCl-phenyl | 1 | 1 |
| 86 | C₃H₅ | H | H | 2,6-DiCl-phenyl | 2 | 1 |
| 87 | C₆H₁₁ | H | H | 2,6-DiCl-phenyl | 1 | 1 |
| 88 | C₆H₁₁ | H | H | 2,6-DiCl-phenyl | 2 | 1 |
| 89 | C₆H₁₁CH₂ | H | H | 2,6-DiCl-phenyl | 1 | 1 |
| 90 | C₆H₁₁CH₂ | H | H | 2,6-DiCl-phenyl | 2 | 1 |
| 91 | C₃H₅CH₂ | H | H | 2,6-DiCl-phenyl | 1 | 1 |
| 92 | C₃H₅CH₂ | H | H | 2,6-DiCl-phenyl | 2 | 1 |
| 93 | CH₃ | CH₃ | CH₃ | 1-CH₃-3-CF₃-5-OCF₂H-pyrazol-4-yl | 1 | 1 |
| 94 | CH₃ | CH₃ | CH₃ | 1-CH₃-3-CF₃-5-OCF₂H-pyrazol-4-yl | 2 | 1 |
| 95 | C₂H₅ | H | H | 1-CH₃-3-CF₃-5-OCF₂H-pyrazol-4-yl | 1 | 1 |
| 96 | C₂H₅ | H | H | 1-CH₃-3-CF₃-5-OCF₂H-pyrazol-4-yl | 2 | 1 |
| 97 | C₂H₅ | H | H | 1-CH₃-3-CF₃-5-OCF₂H-pyrazol-4-yl | 1 | 1 |
| 98 | C₂H₅ | H | H | 1-CH₃-3-CF₃-5-OCF₂H-pyrazol-4-yl | 2 | 1 |
| 99 | C₆H₁₁ | H | H | 1-CH₃-3-CF₃-5-OCF₂H-pyrazol-4-yl | 1 | 1 |
| 100 | C₆H₁₁ | H | H | 1-CH₃-3-CF₃-5-OCF₂H-pyrazol-4-yl | 2 | 1 |
| 101 | C₆H₁₁CH₂ | H | H | 1-CH₃-3-CF₃-5-OCF₂H-pyrazol-4-yl | 1 | 1 |
| 102 | C₆H₁₁CH₂ | H | H | 1-CH₃-3-CF₃-5-OCF₂H-pyrazol-4-yl | 2 | 1 |
| 103 | C₅H₅CH₂ | H | H | 1-CH₃-3-CF₃-5-OCF₂H-pyrazol-4-yl | 1 | 1 |
| 104 | C₅H₅CH₂ | H | H | 1-CH₃-3-CF₃-5-OCF₂H-pyrazol-4-yl | 2 | 1 |
| 105 | CH₃ | H | H | 2-CF₃-4-F-phenyl | 1 | 1 |
| 106 | CH₃ | H | H | 2-CF₃-4-F-phenyl | 2 | 1 |
| 107 | CH₃ | H | H | 2-CF₃-5-F-phenyl | 1 | 1 |
| 108 | CH₃ | H | H | 2-CF₃-5-F-phenyl | 2 | 1 |
| 109 | CH₃ | H | H | 2-CF₃-6-F-phenyl | 1 | 1 |
| 110 | CH₃ | H | H | 2-CF₃-6-F-phenyl | 2 | 1 |
| 111 | CH₃ | H | H | 5-CH₃-1,3,4-thiadiazol-4-yl | 1 | 1 |
| 112 | CH₃ | H | H | 5-CH₃-1,3,4-thiadiazol-4-yl | 2 | 1 |
| 113 | CH₃ | H | H | 2-CH₃-4-CF₃-1,3-thiazol-5-yl | 1 | 1 |
| 114 | CH₃ | H | H | 2-CH₃-4-CF₃-1,3-thiazol-5-yl | 2 | 1 |
| 115 | CH₃ | H | H | 1-CH₃-3-CF₃-5-Cl-pyrazol-4-yl | 1 | 1 |
| 116 | CH₃ | H | H | 1-CH₃-3-CF₃-5-Cl-pyrazol-4-yl | 2 | 1 |
| 117 | *i*C₃H₇ | H | H | 2,6-DiCl-phenyl | 1 | 1 |
| 118 | *i*C₃H₇ | H | H | 2,6-DiCl-phenyl | 2 | 1 |
| 119 | *i*C₃H₇ | H | H | 2,5-DiCl-4-OEt-phenyl | 1 | 1 |
| 120 | *i*C₃H₇ | H | H | 2,5-DiCl-4-OEt-phenyl | 2 | 1 |
| 121 | C₂H₅ | H | H | 1-CH₃-3-CF₃-5-OCH₂CF₃-pyrazol-4-yl | 1 | 1 |
| 122 | C₂H₅ | H | H | 1-CH₃-3-CF₃-5-OCH₂CF₃-pyrazol-4-yl | 2 | 1 |
| 123 | Cl | H | H | 1-CH₃-3-CF₃-5-OCH₂CF₃-pyrazol-4-yl | 1 | 1 |
| 124 | Cl | H | H | 1-CH₃-3-CF₃-5-OCH₂CF₃-pyrazol-4-yl | 2 | 1 |

2. Thiadiazole nach Anspruch 1, die von Verbindungen mit der allgemeinen Formel (I) ausgewählt sind, wobei:
| **Nr.** | **R** | **R₁** | **R₂** | **Y** | **n** | **m** |
|---|---|---|---|---|---|---|
| 1 | CH₃ | H | H | 2,6-DiCl-phenyl | 1 | 1 |
| 51 | CH₃ | H | H | 1-CH₃-3-CF₃-5-OCF₂H-pyrazol-4-yl | 1 | 1 |
| 9 | CH₃ | H | H | 1-CH₃-3-CF₃-5-OCF₂H-pyrazol-4-yl | 1 | 1 |
| 83 | C₂H₅ | H | H | 2,6-DiCl-phenyl | 1 | 1 |
| 95 | C₂H₅ | H | H | 1-CH₃-3-CF₃-5-OCF₂H-pyrazol-4-yl | 1 | 1 |
| 121 | C₂H₅ | H | H | 1-CH₃-3-CF₃-5-OCF₂H-pyrazol-4-yl | 1 | 1 |

3. Herbizide Zusammensetzungen, umfassend: mindestens eine Verbindung mit der Formel (I) nach einem der vorhergehenden Ansprüche, mindestens ein Lösungs- und/oder Verdünnungsmittel, möglicherweise ein oder mehrere agronomisch annehmbare Hilfsstoffe, die ausgewählt sind von: Tensid, Dispergiermittel, Stabilisator und Gemischen davon.

4. Herbizide Zusammensetzungen nach dem vorhergehenden Anspruch, die auch mindestens einen Wirkstoff umfassen, der mit den Verbindungen mit der allgemeinen Formel (I) verträglich ist und ausgewählt ist von: Herbiziden, die sich von denjenigen mit der allgemeinen Formel (I) unterscheiden, Fungiziden, Insektiziden, Akariziden, Düngemitteln und Gemischen davon.

5. Herbizide Zusammensetzungen nach einem oder mehreren der Ansprüche 3 und 4, wobei die Verbindung mit der allgemeinen Formel (I) eine Konzentration in einem Bereich von 1 bis 90 Gew.-% in Bezug auf das Gesamtgewicht der Zusammensetzung aufweist.

6. Verwendung von Thiadiazolen mit der allgemeinen Formel (I) 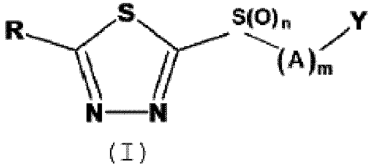 wobei:
- R ein Halogenatom, ein C₁-C₄-Alkyl, ein C₃-C₆-Cycloalkyl oder ein C₄-C₇-Cycloalkylalkyl darstellt;
- A eine Gruppe CR₁R₂ darstellt, wobei R₁ und R₂, die gleich oder unterschiedlich sind, ein Wasserstoffatom, ein C₁-C₄-Alkyl, ein C₃-C₆-Cycloalkyl darstellen;
- Y ein Phenyl, ein Naphthyl, oder eine aromatische heterozyklische Gruppe darstellt, wobei diese Gruppen wahlweise mit mindestens einem Substituenten substituiert sind, der ausgewählt ist von: Halogenatomen, C₁-C₄-Alkyl, C₁-C₄-Haloalkyl, C₁-C₄-Alkoxy, C₁-C₄-Haloalkoxy, C₃-C₆-Cycloalkoxy, C₄-C₇-Cycloalkylalkoxy, Phenoxy, C₁-C₄-Alkylthio, C₁-C₄-Haloalkylthio, C₁-C₄-Alkylsulfinyl, C₁-C₄-Alkylsulfonyl, C₁-C₄-Alkylamino, C₂-C₈-Dialkylamino, Cyan, C₂-C₅-Alkoxycarbonyl, Benzyloxycarbonylen, Phenoxycarbonylen, oder zwei Substituenten zusammen eine C₁-C₄ Alkylendioxy-Gruppe darstellen; wobei im Fall der aromatischen heterozyklischen Gruppe der mindestens eine Substituent auch von Nitro und Carboxyl ausgewählt ist;
- n eine ganze Zahl von 1 bis 2 darstellt;
- m eine ganze Zahl von 1 bis 6 darstellt;
als Herbizide zur Kontrolle von mindestens einem Unkraut in einer landwirtschaftlichen Kultur.

7. Verwendung nach Anspruch 6 zur Kontrolle von mindestens einem Unkraut im Vorlauf oder Nachlauf.

8. Verwendung nach Anspruch 6 oder 7, wobei das Unkraut ausgewählt ist von: Abutilon theofrasti, Alisma plantago, Amaranthus spp., Amni maius, Capsella bursa pastoris, Chenopodium album, Convolvulus sepium, Galium aparine, Geranium dissectum, Heteranthera spp., Ipomea spp., Matricaria spp., Papaver rhoaes, Phaseolus aureus, Polygonum persicaria, Portulaca oleracea, Setaria viridis, Sida spinosa, Sinapsis arvensis, Solanum nigrum, Stellaria media, Veronica spp., Viola spp., Xanthium spp., Alopecurus myosuroides, Anisanta spp., Apera spica-venti, Avena spp., Cyperus spp., Digitaria sanguinalis, Eleusine spp., Echinochloa spp., Eleocharis avicularis, Lolium spp., Panicum spp., Poa spp., Scirpus spp., Sorghum spp..

9. Verwendung nach einem oder mehreren der Ansprüche 6 bis 8, wobei die landwirtschaftliche Kultur von Weizen (Triticum sp.), Gerste (Hordeum vulgare), Mais (Zea mays), Soja (Glycine max) ausgewählt ist.

10. Verfahren zum Kontrollieren von mindestens einem Unkraut in einer landwirtschaftlichen Kultur, das das Anwenden von mindestens einer wirksamen Dosis von mindestens einer Verbindung mit der allgemeinen Formel (I) auf die landwirtschaftliche Kultur umfasst 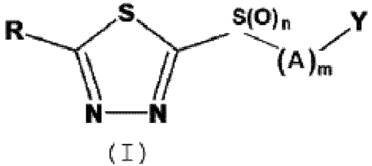 wobei:
- R ein Halogenatom, ein C₁-C₄-Alkyl, ein C₃-C₆-Cycloalkyl oder ein C₄-C₇-Cycloalkylalkyl darstellt;
- A eine Gruppe CR₁R₂ darstellt, wobei R₁ und R₂, die gleich oder unterschiedlich sind, ein Wasserstoffatom, ein C₁-C₄-Alkyl, ein C₃-C₆-Cycloalkyl darstellen;
- Y ein Phenyl, ein Naphthyl oder eine aromatische heterozyklische Gruppe darstellt, wobei diese Gruppen wahlweise mit mindestens einem Substituenten substituiert sind, der ausgewählt ist von: Halogenatomen, C₁-C₄-Alkyl, C₁-C₄-Haloalkyl, C₁-C₄-Alkoxy, C₁-C₄-Haloalkoxy, C₃-C₆-Cycloalkoxy, C₄-C₇-Cycloalkylalkoxy, Phenoxy, C₁-C₄-Alkylthio, C₁-C₄-Haloalkylthio, C₁-C₄-Alkylsulfinyl, C₁-C₄-Alkylsulfonyl, C₁-C₄-Alkylamino, C₂-C₈-Dialkylamino, Cyan, C₂-C₅-Alkoxycarbonyl, Benzyloxycarbonylen, Phenoxycarbonylen, oder die zwei Substituenten zusammen eine C₁-C₄-Alkylendioxy-Gruppe darstellen; wobei im Fall der aromatischen heterozyklischen Gruppe der mindestens eine Substituent auch von Nitro und Carboxyl ausgewählt ist;
- n eine ganze Zahl von 1 bis 2 darstellt;
- m eine ganze Zahl von 1 bis 6 darstellt.

11. Verfahren nach dem vorhergehenden Anspruch, wobei die Verbindung mit der allgemeinen Formel (I) oder die herbizide Zusammensetzung auf die landwirtschaftliche Kultur in einer Dosierung der Verbindung mit der allgemeinen Formel (I) im Bereich von 1 bis 1000 g/ha angewandt wird.

12. Verfahren zur Herstellung von Thiadiazolen mit der allgemeinen Formel (I), das die Phase des Oxidierens einer Verbindung mit der allgemeinen Formel (II) mit mindestens einem Oxidationsmittel in mindestens einem Lösungsmittel gemäß dem folgenden Schema umfasst: 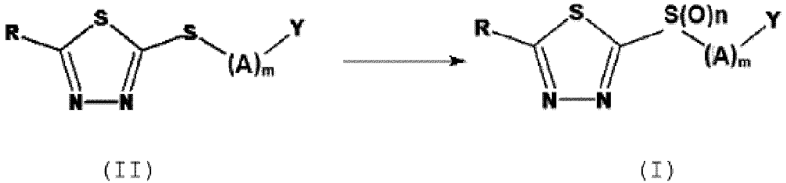 wobei R, R₁, R₂, Y, n und m die in Anspruch 1 definierten Bedeutungen aufweisen.

13. Verfahren nach Anspruch 12, wobei das Oxidationsmittel von einem organischen Peroxid, wie beispielsweise 4-Chlorperbenzoesäure, Peressigsäure oder einem anorganischen Peroxid, wie beispielsweise Wasserstoffperoxid, Kaliumpermanganat, Natriumperiodat ausgewählt wird.

## Revendications

1. Thiadiazoles ayant la formule générale (I), sous forme racémique, isomériquement purs, ou leurs mélanges : 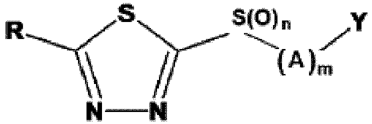 dans laquelle R, R₁, R₂, Y, n et m ont les significations spécifiées ci-dessous :
| **N°** | **R** | **R₁** | **R₂** | **Y** | **n** | **m** |
|---|---|---|---|---|---|---|
| 1 | CH₃ | H | H | 2,6-diCl-phényle | 1 | 1 |
| 2 | CH₃ | H | H | 2,6-diCl-phényle | 2 | 1 |
| 3 | CH₃ | H | H | 2,6-diF-phényle | 1 | 1 |
| 4 | CH₃ | H | H | 2,6-diF-phényle | 2 | 1 |
| 5 | CH₃ | H | H | 2-Cl-6-F-phényle | 1 | 1 |
| 6 | CH₃ | H | H | 2-Cl-6-F-phényle | 2 | 1 |
| 7 | CH₃ | H | H | 2,4-diCl-phényle | 1 | 1 |
| 8 | CH₃ | H | H | 2,4-diCl-phényle | 2 | 1 |
| 9 | CH₃ | H | H | 1-CH₃-3-CF₃-5-OCH₂CF₃-pyrazol-4-yle | 1 | 1 |
| 10 | CH₃ | H | H | 1-CH₃-3-CF₃-5-OCH₂CF₃-pyrazol-4-yle | 2 | 1 |
| 11 | CH₃ | H | H | 2-Cl-4,5-méthyl-endioxy-phényle | 1 | 1 |
| 12 | CH₃ | H | H | 2-Cl-4,5-méthyl-endioxy-phényle | 2 | 1 |
| 13 | CH₃ | H | H | 2,5-diCl-4-OEt-phényle | 1 | 1 |
| 14 | CH₃ | H | H | 2,5-diCl-4-OEt-phényle | 2 | 1 |
| 17 | CH₃ | H | H | 2-CH₃-phényle | 1 | 1 |
| 18 | CH₃ | H | H | 2-CH₃-phényle | 2 | 1 |
| 19 | CH₃ | H | H | 2,5-diCl-4-OMe-phényle | 1 | 1 |
| 20 | CH₃ | H | H | 2,5-diCl-4-OCF₂CF₂H-phényle | 2 | 1 |
| 21 | CH₃ | H | H | 2,5-diCl-4-OCF₂CF₂H-phényle | 1 | 1 |
| 22 | CH₃ | H | H | 2,5-diCl-4-OMe-phényle | 2 | 1 |
| 23 | CH₃ | H | H | 2,5-diCH₃-phényle | 1 | 1 |
| 24 | CH₃ | H | H | 2,5-diCH₃-phényle | 2 | 1 |
| 25 | CH₃ | H | H | 2,6-diOCH₃-phényle | 1 | 1 |
| 26 | CH₃ | H | H | 2,6-diOCH₃-phényle | 2 | 1 |
| 27 | CH₃ | H | H | 3-CF₃-phényle | 1 | 1 |
| 28 | CH₃ | H | H | 2-CF₃-phényle | 2 | 1 |
| 29 | CH₃ | H | H | 2,3,4,5,6-pentaF-phényle | 1 | 1 |
| 30 | CH₃ | H | H | 2,3,4,5,6-pentaF-phényle | 2 | 1 |
| 31 | CH₃ | H | H | 2-SO₂Me-5-COCH₃-6-Cl-phényle | 1 | 1 |
| 32 | CH₃ | H | H | 2-SO₂Me-5-COCH₃-6-Cl-phényle | 2 | 1 |
| 33 | CH₃ | H | H | 2,6-diCl-5-COCH₃-phényle | 1 | 1 |
| 34 | CH₃ | H | H | 2,6-diCl-5-COCH₃-phényle | 2 | 1 |
| 35 | CH₃ | H | H | 2-CH₃-5-Br-thiazol-2-yle | 1 | 1 |
| 36 | CH₃ | H | H | 2-CH₃-5-Br-thiazol-2-yle | 2 | 1 |
| 37 | CH₃ | H | H | 3-COOEt-furan-2-yle | 1 | 1 |
| 38 | CH₃ | H | H | 3-COOEt-furan-2-yle | 2 | 1 |
| 39 | CH₃ | H | H | 3-*t*Bu-isoxazol-2-yle | 1 | 1 |
| 40 | CH₃ | H | H | 3-*t*Bu-isoxazol-2-yle | 2 | 1 |
| 41 | CH₃ | H | H | 6-Cl-pyridin-3-yle | 1 | 1 |
| 42 | CH₃ | H | H | 6-Cl-pyridin-3-yl-N-oxyde | 2 | 1 |
| 43 | CH₃ | H | H | 6-Cl-pyridin-2-yle | 1 | 1 |
| 44 | CH₃ | H | H | 6-Cl-pyridin-2-yl-N-oxyde | 2 | 1 |
| 45 | CH₃ | H | H | pyridin-3-yle | 1 | 1 |
| 46 | CH₃ | H | H | pyridin-3-yl-N-oxyde | 2 | 1 |
| 47 | CH₃ | H | H | pyridin-2-yle | 1 | 1 |
| 48 | CH₃ | H | H | pyridin-2-yl-N-oxyde | 2 | 1 |
| 49 | CH₃ | H | CH₃ | 2,4-diCl-phényle | 1 | 1 |
| 50 | CH₃ | H | CH₃ | 2,4-diCl-phényle | 2 | 1 |
| 51 | CH₃ | H | H | 1-CH₃-3-CF₃-5-OCF₂H-pyrazol-4-yle | 1 | 1 |
| 52 | CH₃ | H | H | 1-CH₃-3-CF₃-5-OCF₂H-pyrazol-4-yle | 2 | 1 |
| 53 | CH₃ | H | H | 1-CH₃-3-CF₂H-pyrazol-4-yle | 1 | 1 |
| 54 | CH₃ | H | H | 1-CH₃-3-CF₂H-pyrazol-4-yle | 2 | 1 |
| 55 | CH₃ | H | H | 4-F-phényle | 1 | 1 |
| 56 | CH₃ | H | H | 4-F-phényle | 2 | 1 |
| 57 | CH₃ | H | H | 1-CH₃-3-CF₃-5-OCF₂H-pyrazol-4-yle | 1 | 2 |
| 58 | CH₃ | H | H | 1-CH₃-3-CF₃-5-OCF₂H-pyrazol-4-yle | 2 | 2 |
| 59 | CH₃ | H | H | 1-CH₃-3-CF₃-5-OCF₂H-pyrazol-4-yle | 1 | 3 |
| 60 | CH₃ | H | H | 1-CH₃-3-CF₃-5-OCF₂H-pyrazol-4-yle | 2 | 3 |
| 63 | CH₃ | H | CH₃ | 1-CH₃-3-CF₃-5-OCF₂H-pyrazol-4-yle | 1 | 1 |
| 64 | CH₃ | H | CH₃ | 1-CH₃-3-CF₃-5-OCF₂H-pyrazol-4-yle | 2 | 1 |
| 65 | CH₃ | H | CH₃ | 1-CH₃-3-CF₃-5-OCF₂H-pyrazol-4-yle | 1 | 2 |
| 66 | CH₃ | H | CH₃ | 1-CH₃-3-CF₃-5-OCF₂H-pyrazol-4-yle | 2 | 2 |
| 67 | CH₃ | H | C₃H₅ | 1-CH₃-3-CF₃-5-OCF₂H-pyrazol-4-yle | 1 | 1 |
| 68 | CH₃ | H | C₃H₅ | 1-CH₃-3-CF₃-5-OCF₂H-pyrazol-4-yle | 2 | 1 |
| 69 | CH₃ | H | H | 2,6-diCl-phényle | 1 | 3 |
| 70 | CH₃ | H | H | 2,6-diCl-phényle | 2 | 3 |
| 71 | CH₃ | H | H | 2,6-diCl-phényle | 1 | 2 |
| 72 | CH₃ | H | H | 2,6-diCl-phényle | 2 | 2 |
| 75 | CH₃ | H | CH₃ | 2,6-diCl-phényle | 1 | 1 |
| 76 | CH₃ | H | CH₃ | 2,6-diCl-phényle | 2 | 1 |
| 77 | CH₃ | H | CH₃ | 2,6-diCl-phényle | 1 | 2 |
| 78 | CH₃ | H | CH₃ | 2,6-diCl-phényle | 2 | 2 |
| 79 | CH₃ | CH₃ | CH₃ | 2,6-diCl-phényle | 1 | 1 |
| 80 | CH₃ | CH₃ | CH₃ | 2,6-diCl-phényle | 2 | 1 |
| 81 | CH₃ | H | C₃H₅ | 2,6-diCl-phényle | 1 | 1 |
| 82 | CH₃ | H | C₃H₅ | 2,6-diCl-phényle | 2 | 1 |
| 83 | C₂H₅ | H | H | 2,6-diCl-phényle | 1 | 1 |
| 84 | C₂H₅ | H | H | 2,6-diCl-phényle | 2 | 1 |
| 85 | C₃H₅ | H | H | 2,6-diCl-phényle | 1 | 1 |
| 86 | C₃H₅ | H | H | 2,6-diCl-phényle | 2 | 1 |
| 87 | C₆H₁₁ | H | H | 2,6-diCl-phényle | 1 | 1 |
| 88 | C₆H₁₁ | H | H | 2,6-diCl-phényle | 2 | 1 |
| 89 | C₆H₁₁CH₂ | H | H | 2,6-diCl-phényle | 1 | 1 |
| 90 | C₆H₁₁CH₂ | H | H | 2,6-diCl-phényle | 2 | 1 |
| 91 | C₃H₅CH₂ | H | H | 2,6-diCl-phényle | 1 | 1 |
| 92 | C₃H₅CH₂ | H | H | 2,6-diCl-phényle | 2 | 1 |
| 93 | CH₃ | CH₃ | CH₃ | 1-CH₃-3-CF₃-5-OCF₂H-pyrazol-4-yle | 1 | 1 |
| 94 | CH₃ | CH₃ | CH₃ | 1-CH₃-3-CF₃-5-OCF₂H-pyrazol-4-yle | 2 | 1 |
| 95 | C₂H₅ | H | H | 1-CH₃-3-CF₃-5-OCF₂H-pyrazol-4-yle | 1 | 1 |
| 96 | C₂H₅ | H | H | 1-CH₃-3-CF₃-5-OCF₂H-pyrazol-4-yle | 2 | 1 |
| 97 | C₂H₅ | H | H | 1-CH₃-3-CF₃-5-OCF₂H-pyrazol-4-yle | 1 | 1 |
| 98 | C₂H₅ | H | H | 1-CH₃-3-CF₃-5-OCF₂H-pyrazol-4-yle | 2 | 1 |
| 99 | C₆H₁₁ | H | H | 1-CH₃-3-CF₃-5-OCF₂H-pyrazol-4-yle | 1 | 1 |
| 100 | C₆H₁₁ | H | H | 1-CH₃-3-CF₃-5-OCF₂H-pyrazol-4-yle | 2 | 1 |
| 101 | C₆H₁₁CH₂ | H | H | 1-CH₃-3-CF₃-5-OCF₂H-pyrazol-4-yle | 1 | 1 |
| 102 | C₆H₁₁CH₂ | H | H | 1-CH₃-3-CF₃-5-OCF₂H-pyrazol-4-yle | 2 | 1 |
| 103 | C₅H₅CH₂ | H | H | 1-CH₃-3-CF₃-5-OCF₂H-pyrazol-4-yle | 1 | 1 |
| 104 | C₅H₅CH₂ | H | H | 1-CH₃-3-CF₃-5-OCF₂H-pyrazol-4-yle | 2 | 1 |
| 105 | CH₃ | H | H | 2-CF₃-4-F-phényle | 1 | 1 |
| 106 | CH₃ | H | H | 2-CF₃-4-F-phényle | 2 | 1 |
| 107 | CH₃ | H | H | 2-CF₃-5-F-phényle | 1 | 1 |
| 108 | CH₃ | H | H | 2-CF₃-5-F-phényle | 2 | 1 |
| 109 | CH₃ | H | H | 2-CF₃-6-F-phényle | 1 | 1 |
| 110 | CH₃ | H | H | 2-CF₃-6-F-phényle | 2 | 1 |
| 111 | CH₃ | H | H | 5-CH₃-1,3,4-thiadiazol-4-yle | 1 | 1 |
| 112 | CH₃ | H | H | 5-CH₃-1,3,4-thiadiazol-4-yle | 2 | 1 |
| 113 | CH₃ | H | H | 2-CH₃-4-CF₃-1,3-thiazol-5-yle | 1 | 1 |
| 114 | CH₃ | H | H | 2-CH₃-4-CF₃-1,3-thiazol-5-yle | 2 | 1 |
| 115 | CH₃ | H | H | 1-CH₃-3-CF₃-5-Cl-pyrazol-4-yle | 1 | 1 |
| 116 | CH₃ | H | H | 1-CH₃-3-CF₃-5-Cl-pyrazol-4-yle | 2 | 1 |
| 117 | *i*C₃H₇ | H | H | 2,6-diCl-phényle | 1 | 1 |
| 118 | *i*C₃H₇ | H | H | 2,6-diCl-phényle | 2 | 1 |
| 119 | *i*C₃H₇ | H | H | 2,5-diCl-4-OEt-phényle | 1 | 1 |
| 120 | *i*C₃H₇ | H | H | 2,5-diCl-4-OEt-phényle | 2 | 1 |
| 121 | C₂H₅ | H | H | 1-CH₃-3-CF₃-5-OCH₂CF₃-pyrazol-4-yle | 1 | 1 |
| 122 | C₂H₅ | H | H | 1-CH₃-3-CF₃-5-OCH₂CF₃-pyrazol-4-yle | 2 | 1 |
| 123 | Cl | H | H | 1-CH₃-3-CF₃-5-OCH₂CF₃-pyrazol-4-yle | 1 | 1 |
| 124 | Cl | H | H | 1-CH₃-3-CF₃-5-OCH₂CF₃-pyrazol-4-yle | 2 | 1 |

2. Thiadiazoles selon la revendication 1, sélectionnés parmi les composés ayant la formule générale (I), dans laquelle :
| **N°** | **R** | **R₁** | **R₂** | **Y** | **n** | **m** |
|---|---|---|---|---|---|---|
| 1 | CH₃ | H | H | 2,6-diCl-phényle | 1 | 1 |
| 51 | CH₃ | H | H | 1-CH₃-3-CF₃-5-OCF₂H-pyrazol-4-yle | 1 | 1 |
| 9 | CH₃ | H | H | 1-CH₃-3-CF₃-5-OCF₂H-pyrazol-4-yle | 1 | 1 |
| 83 | C₂H₅ | H | H | 2,6-diCl-phényle | 1 | 1 |
| 95 | C₂H₅ | H | H | 1-CH₃-3-CF₃-5-OCF₂H-pyrazol-4-yle | 1 | 1 |
| 121 | C₂H₅ | H | H | 1-CH₃-3-CF₃-5-OCF₂H-pyrazol-4-yle | 1 | 1 |

3. Compositions herbicides comprenant au moins un composé ayant la formule (I) selon l'une quelconque des revendications précédentes, au moins un solvant et/ou diluant, éventuellement un ou plusieurs excipients acceptables en agronomie sélectionnés parmi : un tensioactif, un dispersant, un stabilisant et leurs mélanges.

4. Compositions herbicides selon la revendication précédente comprenant également au moins une substance active compatible avec les composés ayant la formule générale (I) sélectionnée parmi : des herbicides différents de ceux ayant la formule générale (I), des fongicides, des insecticides, des acaricides, des engrais et leurs mélanges.

5. Compositions herbicides selon l'une ou plusieurs des revendications 3 à 4, dans lesquelles ledit composé ayant la formule générale (I) possède une concentration allant de 1 % à 90 % en poids par rapport au poids total de la composition.

6. Utilisation de thiadiazoles ayant la formule générale (I) 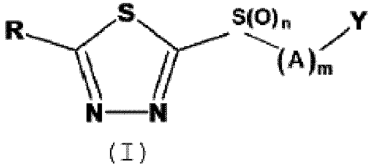 dans laquelle :
- R représente un atome d'halogène, un alkyle en C₁ à C₄, un cycloalkyle en C₃ à C₆ ou un cycloalkylalkyle en C₄ à C₇ ;
- A représente un groupe CR₁R₂, dans lequel R₁ et R₂, identiques ou différents, représentent un atome d'hydrogène, un alkyle en C₁ à C₄, un cycloalkyle en C₃ à C₆ ;
- Y représente un groupe phényle, naphtyle ou hétérocyclique aromatique, ces groupes étant facultativement substitués avec au moins un substituant sélectionné parmi : des atomes d'halogène, un alkyle en C₁ à C₄, un haloalkyle en C₁ à C₄, un alkoxy en C₁ à C₄, un haloalkoxy en C₁ à C₄, un cycloalkoxy en C₃ à C₆, un cycloalkylalkoxy en C₄ à C₇, un phénoxy, un alkylthio en C₁ à C₄, un haloalkylthio en C₁ à C₄, un alkylsulfinyle en C₁ à C₄, un alkylsulfonyle, un alkylamino en C₁ à C₄, un dialkylamino en C₂ à C₈, un cyano, un alkoxycarbonyle en C₂ à C₅, des benzyloxycarbonyles, des phénoxycarbonyles, ou deux substituants qui représentent conjoinement un groupe alkylènedioxy en C₁ à C₄ ; dans le cas du groupe hétérocyclique aromatique, ledit au moins un substituant étant également sélectionné parmi les groupes nitro et carboxyle ;
- n représente un nombre entier de 1 à 2 ;
- m représente un nombre entier de 1 à 6,
comme herbicides pour la lutte contre au moins une mauvaise herbe dans une culture agricole.

7. Utilisation selon la revendication 6 pour la lutte contre au moins une mauvaise herbe en pré-levée ou en post-levée.

8. Utilisation selon la revendication 6 ou 7, dans laquelle ladite mauvaise herbe est sélectionnée parmi : Abutilon theofrasti, Alisma plantago, Amaranthus spp., Amni maius, Capsella bursa pastoris, Chenopodium album, Convolvulus sepium, Galium aparine, Geranium dissectum, Heteranthera spp., Ipomea spp., Matricaria spp., Papaver rhoaes, Phaseolus aureus, Polygonum persicaria, Portulaca oleracea, Setaria viridis, Sida spinosa, Sinapsis arvensis, Solanum nigrum, Stellaria média, Veronica spp., Viola spp., Xanthium spp., Alopecurus myosuroides, Anisanta spp., Apera spica-venti, Avena spp., Cyperus spp., Digitaria sanguinalis, Eleusine Eleocharis avicularis, spp. Echinochloa spp., Lolium spp., Panicum spp., Poa spp., Scirpus spp., Sorghum spp..

9. Utilisation selon l'une ou plusieurs des revendications 6 à 8, dans laquelle ladite culture agricole est sélectionnée parmi le blé (Triticum sp.), l'orge (Hordeum vulgare), le maïs (Zea mays), le soya (Glycine max).

10. Procédé de lutte contre au moins une mauvaise herbe dans une culture agricole qui comprend l'application à la culture agricole d'au moins une dose efficace d'au moins un composé ayant la formule générale (I) 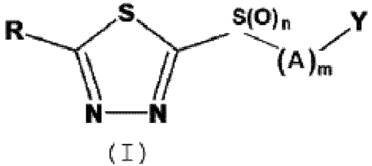 dans laquelle :
- R représente un atome d'halogène, un alkyle en C₁ à C₄, un cycloalkyle en C₃ à C₆ ou un cycloalkylalkyle en C₄ à C₇ ;
- A représente un groupe CR₁R₂, dans lequel R₁ et R₂, identiques ou différents, représentent un atome d'hydrogène, un alkyle en C₁ à C₄, un cycloalkyle en C₃ à C₆ ;
- Y représente un groupe phényle, naphtyle ou hétérocyclique aromatique, ces groupes étant facultativement substitués avec au moins un substituant sélectionné parmi : des atomes d'halogène, un alkyle en C₁ à C₄, un haloalkyle en C₁ à C₄, un alkoxy en C₁ à C₄, un haloalkoxy en C₁ à C₄, un cycloalkoxy en C₃ à C₆, un cycloalkylalkoxy en C₄ à C₇, un phénoxy, un alkylthio en C₁ à C₄, un haloalkylthio en C₁ à C₄, un alkylsulfinyle en C₁ à C₄, un alkylsulfonyle en C₁ à C₄, un alkylamino en C₁ à C₄, un dialkylamino en C₂ à C₈, un cyano, un alkoxycarbonyle en C₂ à C₅, des benzyloxycarbonyles, des phénoxycarbonyles, ou deux substituants qui représentent conjoinement un groupe alkylènedioxy en C₁ à C₄ ; dans le cas du groupe hétérocyclique aromatique, ledit au moins un substituant étant également sélectionné parmi les groupes nitro et carboxyle ;
- n représente un nombre entier de 1 à 2 ;
- m représente un nombre entier de 1 à 6.

11. Procédé selon la revendication précédente, dans lequel ledit composé ayant la formule générale (I) ou ladite composition herbicide est appliqué(e) à la culture agricole dans un dosage dudit composé ayant la formule générale (I) dans la plage de 1 à 1000 g/ha.

12. Processus de préparation de thiadiazoles ayant la formule générale (I) comprenant la phase d'oxydation d'un composé ayant la formule générale (II) avec au moins un agent oxydant dans au moins un solvant, selon le schéma 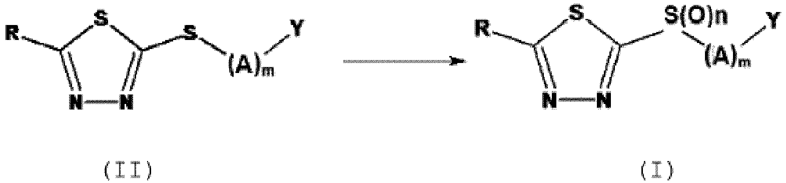 dans lequel R, R₁, R₂, Y, n et m ont les significations définies selon la revendication 1.

13. Processus selon la revendication 12, dans lequel ledit agent oxydant est sélectionné parmi un peroxyde organique, tel que l'acide 4-chloro-perbenzoïque, l'acide peracétique ou un peroxyde inorganique, tel que le peroxyde d'hydrogène, le permanganate de potassium, le periodate de sodium.
